# EUROPEAN PATENT SPECIFICATION

(11) **EP 4 359 374 B1**
(45) Date of publication and mention of the grant of the patent: **19.03.2025**
(21) Application number: 22733380.4
(22) Date of filing: 10.06.2022
(51) Int. Cl.: C07C 29/42, C07C 33/042

(54) **PROCESS FOR THE SYNTHESIS OF PROPARGYLIC ALCOHOL BY REACTING FORMALDEHYDE WITH ACETYLENE IN THE PRESENCE OF A HOMOGENEOUS COPPER CATALYST**
VERFAHREN ZUR SYNTHESE VON PROPARGYLALKOHOL DURCH UMSETZUNG VON FORMALDEHYD MIT ACETYLEN IN GEGENWART EINES HOMOGENEN KUPFERKATALYSATORS
PROCÉDÉ DE SYNTHÈSE D'ALCOOL PROPARGYLIQUE PAR RÉACTION DE FORMALDÉHYDE AVEC DE L'ACÉTYLÈNE EN PRÉSENCE D'UN CATALYSEUR AU CUIVRE HOMOGÈNE

(30) Priority: 21.06.2021 EP 21180528
(43) Date of publication of application: 01.05.2024
(73) Proprietor: BASF SE, 67056 Ludwigshafen am Rhein (DE)
(72) Inventor: SCHAUB, Thomas, 67056 Ludwigshafen (DE); SHEVCHENKO, Grigory Andre, 67056 Ludwigshafen (DE); HASHMI, A. Stephen K., 69117 Heidelberg (DE); SITTE, Nikolai Amadeus, 69117 Heidelberg (DE)
(74) Representative: BASF IP Association
(86) International application number: PCT/EP2022/065792
(87) International publication number: WO 2022/268526

(56) References cited:
- EP-A1- 0 305 688
- EP-A1- 1 658 256
- TROTUS IOAN-TEODOR ET AL: "Catalytic Reactions of Acetylene: A Feedstock for the Chemical Industry Revisited", CHEMICAL REVIEWS, vol. 114, no. 3, 12 February 2014 (2014-02-12), US, pages 1761 - 1782, XP055972922, ISSN: 0009-2665, DOI: 10.1021/cr400357r
- KUNDU SHRISHNU KUMAR ET AL: "Copper (I) iodide catalyzed synthesis of primary propargylic alcohols from terminal alkyne", RSC ADVANCES, vol. 5, no. 17, 1 January 2015 (2015-01-01), GB, pages 13220 - 13223, XP055972991, ISSN: 2046-2069, DOI: 10.1039/C4RA12719F
- SITTE NIKOLAI A. ET AL: "Copper-Catalysed Synthesis of Propargyl Alcohol and Derivatives from Acetylene and other Terminal Alkynes", ADVANCED SYNTHESIS AND CATALYSIS, vol. 364, no. 13, 5 July 2022 (2022-07-05), pages 2227 - 2234, XP055972862, ISSN: 1615-4150, Retrieved from the Internet <URL:https://onlinelibrary.wiley.com/doi/full-xml/10.1002/adsc.202200369> DOI: 10.1002/adsc.202200369

## Description

Object of the invention is a process to produce propargylic alcohol by homogeneous catalysis, wherein acetylene is reacted with formaldehyde in the presence of a copper complex comprising at least one phosphine as ligand.

Propargylic alcohol is currently produced from acetylene and formaldehyde using heterogeneous copper acetylide catalysts, but the selectivity is usually relatively low and significant amounts of 1,4.butynediol are formed as by-product. Therefore, more selective synthetic methods are required for an economic synthesis of propargylic alcohol from acetylene and formaldehyde to cover the growing market demand for propargylic alcohol.

Justus Liebigs Annalen der Chemie, 1955, 596, 25-38 describes the reaction of aqueous formaldehyde with acetylene at a pressure of 20 bar using a heterogeneous copper acetylide catalyst and tetrahydrofurane as solvent at 100°C. After isolation of the products, 62% propargylic alcohol and 30% 1,4-butynediol (according the used formaldehyde) can be obtained (ratio propargylic alcohol : 1,4-butynediol = 2.1 : 1). Drawbacks of this approach are the 30% of unwanted 1,4-butynediol and the necessary high acetylene pressure of 20 bar, which requires high safety measures.

US 2996552A discloses the use of acetylene at atmospheric pressure, 10% KOH as a catalysts, dimethylsulfoxide as the solvent and solid paraformaldehyde as the formaldehyde source for the synthesis of propargylic alcohol. A conversion of the paraformaldehyde of 40% to the propargylic alcohol and of 9% to the 1,4-butynediol (ratio propargylic alcohol : 1,4-butyendiol = 4.4 : 1). Drawbacks of this approach are the low conversion of the paraformaldehyde, the amount of unwanted 1,4-butynediol formed as well as the necessary neutralization of the catalyst at the end of the reaction, which therefore cannot be recycled and causes salt waste.

DE 4415380 discloses the reaction of acetylene at a pressure of maximum 1.4 bar with aqueous formaldehyde using a heterogeneous copper acetylide catalyst and dimethylformamide as the solvent. A conversion of the paraformaldehyde up to 75% towards propargylic alcohol and 1,4-butynediol is disclosed with a maximum ratio of propargylic alcohol to 1,4-butyendiol of 3 : 1. A drawback of this approach is the still relative high amount of unwanted 1,4-butynediol formed.

EP1658256 discloses the reaction of acetylene at a pressure of 3.3 bar with aqueous formaldehyde using a heterogeneous copper acetylide catalyst in a moving bed using tetrahydrofurane as solvent. The highest ratio of propargylic alcohol to 1,4-butyendiol obtained is 0.17 : 1. A drawback of this approach is the high amount of unwanted 1,4-butynediol formed, which is in this process the main product.

For the synthesis of other alkynols based on terminal alkynes certain transition metal catalyzed systems are known.

In Organic Letters, 2005, 7, 4395-4398, a homogeneously dissolved silver catalyst with a phosphine ligand and a trialkylamine as additive is used in water as the solvent to synthesize a series of alkynols from terminal alkynes like phenylacetylene and different aldehydes. Drawbacks of this system are, that it could not be shown, that it works with acetylene and formaldehyde in a selective manner to obtain propargylic alcohol and silver could form in this case the highly explosive silver acetylide, when trying to use this system for the reaction of acetylene.

In the Journal of Organic Chemistry, 2007, 72, 9560-9596, a homogeneously dissolved rhodium catalyst with a phosphine ligand is s used in dioxane as the solvent to synthesize a series of alkynols from terminal alkynes and different ketones and aldehydes. Drawbacks of this system are, that it could not be shown, that it works with acetylene and formaldehyde in a selective manner to obtain propargylic alcohol and that the very expensive precious metal rhodium is used for the catalyst.

In RSC Advances, 2015, 5, 13220-13223, copper(I)iodide is used in the presence of one equivalent of an inorganic base and one equivalent of a trialkylamine base in dimethylsulfoxide as solvent to synthesize a series of alkynols from terminal alkynes an paraformaldehyde. It is described in this work, that in the presence of a phosphine (PPh₃) as ligand, no propargylic alcohol is formed under the given conditions, instead, only dimerization of the alkyne occurs. Drawbacks of this system are, that it could not be shown, that it works with acetylene and formaldehyde in a selective manner to obtain propargylic alcohol and also that stoichiometric amounts of bases as additives are necessary, which causes higher production costs and waste.

It was an object of this invention to provide a process for the synthesis of propargylic alcohol from formaldehyde and acetylene, which can be performed at low acetylene pressures and with a high selectivity to propargylic alcohol without the use of other stoichiometric co-reagents.

Accordingly, a process to produce propargylic alcohol has been found, wherein acetylene is reacted with formaldehyde in the liquid phase in the presence of a copper catalyst and at least one phosphine.

Preferably this process is performed with a homogeneous copper catalyst having at least one phosphine as ligand.

### Formaldehyde

Formaldehyde can be used in different forms for the disclosed process. It can be used in the process as technical aqueous solution with a formaldehyde content of up to 50wt%, as a solution in different alcohols as in methanol (up to 55 wt% formaldehyde), butanol (up to 40 wt% formaldehyde), as paraformaldehyde, trioxane, acetals (e.g. dimethoxymethane, diethoxymethane) or as pure gaseous formaldehyde.

### Copper Catalyst

A homogeneous copper catalyst Cu(I) can be employed in the form of a preformed copper complex which comprises the copper, the required phosphine ligand(s) and one or more other ligands. Alternatively, the catalytic system is formed in situ in the reaction mixture by combining a copper compound, herein also termed pre-catalyst, with one or more suitable phosphine ligands to form a catalytically active copper complex in the reaction mixture.

Suitable pre-catalysts are selected from neutral copper complexes, oxides and salts of copper. Copper compounds that are useful as pre-catalyst are, for example, [CuBr(Me₂S)], [Cu(CF₃SO₃)₂], [CuBr], [CuCl], [Cul], [CuF], [Cu(OAc)₂], [Cu(OAc)₂]*H₂O, [Cu(OAc)], [(PPh₃)₂Cu][NO₃], [Cu(AcAc)₂], [Cu((CH₃)₂CHCOO)₂], [CuCl₂], [CuCl₂]*2H₂O, [Cu(CN)], [Cu(HCOO)₂]*(H₂O)ₓ, [Cu(OCH₃)₂], [Cu(CO₃)₂]*3H₂O, [Cu(O₂CCO₂)]*0.5H₂O, [Cu(ClO₄)₂]*6H₂O, [Cu(CCPh)], [Cu₂C₂]*H₂O [Cu(SO₄)], [Cu(SO₄)]*5H₂O, [Cu(NO₃)₂], [Cu(BF₄)₂]*H₂Oₓ, [Cu(SCN)], [Cu(CF₃SO₃)₂], [Cu(CH₃CN)₄][PF₆], [Cu(CH₃CN)₄][BF₄], [Cu(CH₃CN)₄][NO₃] and [Cu(CH₃CN)₄][ClO₄].

For the alkynylation of the process according the present invention, a suitable phosphine ligand must be employed in combination with copper.

Suitable phosphine ligands of the catalytic system for the alkynylation of formaldehyde in the process according to the invention are, for example, mono-, bi-, tri- and tetra dentate phosphines of the formulae I and II shown below, where 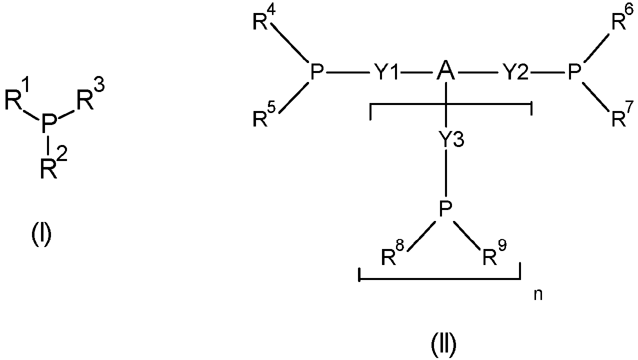
- n: is 0 or 1;
- R¹ to R⁹: are, independently of one another, unsubstituted or at least monosubstituted C₁-C₁₀-alkyl, C₃-C₁₀-cycloalkyl, C₃-C₁₀-heterocyclyl comprising at least one heteroatom selected from N, O and S, C₅-C₁₄-aryl or C₅-C₁₀-heteroaryl comprising at least one heteroatom selected from N, O and S,
where the substituents are selected from the group consisting of: F, Cl, Br, OH, CN, NH₂ and C₁-C₁₀-alkyl;
- A: is
i) a bridging group selected from the group unsubstituted or at least monosubstituted N, O, P, C₁-C₆-alkane, C₃-C₁₀-cycloalkane, C₃-C₁₀-heterocycloalkane comprising at least one heteroatom selected from N, O and S, C₅-C₁₄-aromatic and C₅-C₆-heteroaromatic comprising at least one heteroatom selected from N, O and S,
where the substituents are selected from the group consisting of:
C₁-C₄-alkyl, phenyl, F, Cl, Br, OH, OR¹⁵, NH₂, NHR¹⁵ or N(R¹⁵)₂,
where R¹⁵ is selected from C₁-C₁₀-alkyl and C₅-C₁₀-aryl;
   or
   ii) a bridging group of the formula (III) or (IV): 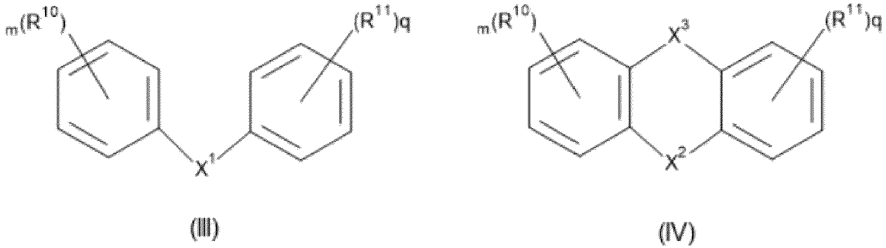
   - m, q: are, independently of one another, 0, 1, 2, 3 or 4;
   - R¹⁰, R¹¹: are, independently of one another, selected from the group C₁-C₁₀-alkyl, F, Cl, Br, OH, OR¹⁸, NH₂, NHR¹⁸ and N(R¹⁸)₂,
   where R¹⁸ is selected from C₁-C₁₀-alkyl and C₅-C₁₀-aryl;
   - X¹, X²: are, independently of one another, NH, O or S;
   - X³: is a bond, NH, NR¹⁷, O, S or CR¹⁸R¹⁹;
   - R¹⁷: is unsubstituted or at least monosubstituted C₁-C₁₀-alkyl, C₃-C₁₀-cycloalkyl, C₃-C₁₀-heterocyclyl comprising at least one heteroatom selected from N, O and S, C₅-C₁₄-aryl or C₅-C₁₀-heteroaryl comprising at least one heteroatom selected from N, O and S,
   where the substituents are selected from the group consisting of: F, Cl, Br, OH, CN, NH₂ and C₁-C₁₀-alkyl;
   - R¹⁸, R¹⁹: are, independently of one another, unsubstituted or at least monosubstituted C₁-C₁₀-alkyl, C₁-C₁₀-alkoxy, C₃-C₁₀-cycloalkyl, C₃-C₁₀-cycloalkoxy, C₃-C₁₀-heterocyclyl comprising at least one heteroatom selected from N, O and S, C₅-C₁₄-aryl, C₅-C₁₄-aryloxy or C₅-C₁₀-heteroaryl comprising at least one heteroatom selected from N, O and S,
   where the substituents are selected from the group consisting of: F, Cl, Br, OH, CN, NH₂ and C₁-C₁₀-alkyl;
   - Y¹, Y², Y³: are, independently of one another, a bond, unsubstituted or at least monosubstituted methylene, ethylene, trimethylene, tetramethylene, pentamethylene or hexamethylene, where the substituents are selected from the group consisting of: F, Cl, Br, OH, OR¹⁵, CN, NH₂, NHR¹⁶, N(R¹⁶)₂ and C₁-C₁₀-alkyl, where R¹⁶ is selected from C₁-C₁₀-alkyl and C₅-C₁₀-aryl.
A is a bridging group

For the case that A is selected from the group unsubstituted or at least monosubstituted C₁-C₆-alkane, C₃-C₁₀-cycloalkane, C₃-C₁₀-heterocycloalkane, C₅-C₁₄-aromatic and C₅-C₆-heteroaromatic for the case (n = 0), two hydrogen atoms of the bridging group are replaced by bonds to the adjacent substituents Y¹ and Y². For the case (n = 1), three hydrogen atoms of the bridging group are replaced by three bonds to the adjacent substituents Y¹, Y² and Y³.

For the case that A is P (phosphorus), the phosphorus forms for the case (n = 0) two bonds to the adjacent substituents Y¹ and Y² and one bond to a substituent selected from the group consisting of C₁-C₄-alkyl and phenyl. For the case (n = 1), the phosphorus forms three bonds to the adjacent substituents Y¹, Y² and Y³.

For the case that A is N (nitrogen), the nitrogen for the case (n = 0) forms two bonds to the adjacent substituents Y¹ and Y² and one bond to a substituent selected from the group consisting of C₁-C₄-alkyl and phenyl. For the case (n = 1), the nitrogen forms three bonds to the adjacent substituents Y¹, Y² and Y³.

For the case that A is O (oxygen), n = 0. The oxygen forms two bonds to the adjacent substituents Y¹ and Y².

In a preferred embodiment, the process according to the invention is carried out in the presence of a copper complex catalyst and also at least one phosphorus donor ligand of the general formula (II).

In a preferred embodiment, the process according to the invention is carried out in the presence of at least one copper complex catalyst and at least one phosphorus donor ligand of the general formula (V), 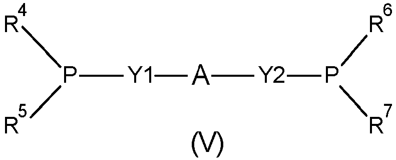 where
- R⁴ to R⁷: are, independently of one another, unsubstituted or at least monosubstituted C₁-C₁₀-alkyl, C₃-C₁₀-cycloalkyl, C₃-C₁₀-heterocyclyl comprising at least one heteroatom selected from N, O and S, C₅-C₁₄-aryl or C₅-C₁₀-heteroaryl comprising at least one heteroatom selected from N, O and S,
where the substituents are selected from the group consisting of: F, Cl, Br, OH, CN, NH₂ and C₁-C₁₀-alkyl;
- A: is
i) a bridging group selected from the group unsubstituted or at least monosubstituted N, O, P, C₁-C₆-alkane, C₃-C₁₀-cycloalkane, C₃-C₁₀-heterocycloalkane comprising at least one heteroatom selected from N, O and S, C₅-C₁₄-aromatic and C₅-C₆-heteroaromatic comprising at least one heteroatom selected from N, O and S,
where the substituents are selected from the group consisting of:
C₁-C₄-alkyl, phenyl, F, Cl, Br, OH, OR¹⁶, NH₂, NHR¹⁶ or N(R¹⁶)₂,
where R¹⁶ is selected from C₁-C₁₀-alkyl and C₅-C₁₀-aryl;

In an embodiment, the process according to the invention is carried out in the presence of a copper complex catalyst and monodentate ligands of the formula I are preferred herein are those in which R¹, R² and R³ are each phenyl or alkyl optionally carrying 1 or 2 C₁-C₄-alkyl substituents and those in which R¹, R² and R³ are each C₅-C₈-cycloalkyl or C₂-C₁₀-alkyl. The groups R¹ to R³ may be different or identical. Preferably the groups R^{5a} to R⁶ are identical and are selected from the substituents mentioned herein, in particular from those indicated as preferred. Examples of preferable monodentate ligands I are triphenylphosphine (TPP), Triethylphosphine, tri-n-butylphosphine, tri-n-octylphosphine, tritertbutylphosphine and tricyclohexylphosphine, triadamantylphosphine, diadamantyl-n-butyl-phosphine.

In another embodiment, the process according to the invention is carried out in the presence of a copper complex catalyst and at least a bidentate ligand of the formula V ligand selected from the group consisting of 1,2-bis(diphenylphosphino)ethane (dppe), 1,3-bis(diphenylphosphino)propane (dppp), 1,4-bis(diphenylphosphino)butane (dppb), 1,2-bis(dicyclohexylphosphino)ethane (dcpe), 1,3-bis(dicyclohexylphosphino)propane (dcpp), 1,4-bis(dicyclohexylphosphino)butane (dcpb), 1,2-bis(ditertbutylphosphino)ethane (dtpe), 1,3-bis(ditertbutylphosphino)propane (dtpp), 1,4-bis(ditertbutylphosphino)butane (dtpb), 1,2-bis(diisopropylphosphino)ethane (dcpe), 1,3-bis(diisopropylphosphino)propane (dcpp), 1,4-bis(diisopropyllphosphino)butane (dcpb), 1,2-bis(diphenylphosphino)benzene, 1,2-bis(dicyclohexylphosphino)benzene, 1,2-bis(diisopropylphosphino)benzene, 1,2-bis(dtertbutylphosphino)benzene, 1,2-bis-(diphenylphosphino-methylene)benzene, 1,2-bis-(diisopropylphosphino-methylene)benzene, 1,2-bis-(ditertbutylphosphino-methylene)benzene, 1,2-bis-(dicyclohexyl)ylphosphino-methylene)benzene , in particular 1,4-bis(dicyclohexylphosphino)butane (dcpb) and 1,2-bis-(ditertbutylphosphino-methylene)benzene

Within the context of the present invention, C₁-C₁₀-alkyl is understood as meaning branched, unbranched, saturated and unsaturated groups. Preference is given to alkyl groups having 1 to 6 carbon atoms (C₁-C₆-alkyl). More preference is given to alkyl groups having 1 to 4 carbon atoms (C₁-C₄-alkyl).

Examples of saturated alkyl groups are methyl, ethyl, n-propyl, isopropyl, n-butyl, isobutyl, sec-butyl, tert-butyl, amyl and hexyl.

Examples of unsaturated alkyl groups (alkenyl, alkynyl) are vinyl, allyl, butenyl, ethynyl and propynyl.

The C₁-C₁₀-alkyl group can be unsubstituted or substituted with one or more substituents selected from the group F, Cl, Br, hydroxy (OH), C₁-C₁₀-alkoxy, C₅-C₁₀-aryloxy, C₅-C₁₀-alkylaryloxy, C₅-C₁₀-heteroaryloxy comprising at least one heteroatom selected from N, O, S, oxo, C₃-C₁₀-cycloalkyl, phenyl, C₅-C₁₀-heteroaryl comprising at least one heteroatom selected from N, O, S, C₅-C₁₀-heterocyclyl comprising at least one heteroatom selected from N, O, S, naphthyl, amino, C₁-C₁₀-alkylamino, C₅-C₁₀-arylamino, C₅-C₁₀-heteroarylamino comprising at least one heteroatom selected from N, O, S, C₁-C₁₀-dialkylamino, C₁₀-C₁₂-diarylamino, C₁₀-C₂₀-alkylarylamino, C₁-C₁₀-acyl, C₁-C₁₀-acyloxy, NO₂, C₁-C₁₀-carboxy, carbamoyl, carboxamide, cyano, sulfonyl, sulfonylamino, sulfinyl, sulfinylamino, thiol, C₁-C₁₀-alkylthiol, C₅-C₁₀-arylthiol or C₁-C₁₀-alkylsulfonyl.

The above definition for C₁-C₁₀-alkyl applies correspondingly to C₁-C₃₀-alkyl and to C₁-C₆-alkane.

C₃-C₁₀-cycloalkyl is understood in the present case as meaning saturated, unsaturated monocyclic and polycyclic groups. Examples of C₃-C₁₀-cycloalkyl are cyclopropyl, cyclobutyl, cyclopentyl, cyclohexyl or cycloheptyl. The cycloalkyl groups can be unsubstituted or substituted with one or more substituents as has been defined above in connection with the group C₁-C₁₀-alkyl.

The active copper catalyst can be generated in situ in the reaction mixture by adding the ligands to the above-mentioned precursors. The molar ratio between the copper metal and the ligand is in the range of 2 : 1 to 1 : 50, preferable in the range of 1 : 1 to 1 : 10 most preferably in the range of 1 : 2 to 1 : 5.

The active copper catalyst can also be generated in a step before the alkynylation reaction by adding the ligands to the above-mentioned precursors in an appropriate organic solvent providing a mixing gap with water as for example benzene, toluene, xylenes, chlorobenzene or chloroform at temperatures between 25°C and 120°C for 10 minutes to 10 hours. In one embodiment, acetylene is present in this catalyst activation step. The acetylene is either added dissolved in the organic solvent or pressurized to the mixture in the activation at a pressure between 1 bar and 20 bar. In one embodiment, insoluble material after the catalyst generation, if formed, is filtered of. The obtained solutions with the active catalyst, is then used in the alkynylation reaction.

In addition to the one or more ligands selected from the groups of ligands described above the catalytic system of the inventive process may also include at least one further ligand which is selected from halides, alkynes, amides, carboxylates, acetylacetonate, aryl- or alkylsufonates, hydride, CO, olefins, dienes, cycloolefins, nitriles, aromatics and heteroaromatics, ethers, PF₃, phospholes, phosphabenzenes, and mono-, di- and polydentate phosphinite, phosphonite, phosphoramidite and phosphite ligands.

The active catalyst can also be preformed in a dedicated synthetic step. Appropriate preformed catalysts can be [Cu₂(dcpb)₃][PF₆]₂, [Cu₂(dcpb)₃][NO₃]₂, [Cu₂(dcpb)₃][BF₄]₂, [Cu₂(dcpb)₃][ClO₄]₂ [Cu₂(dppe)₃][PF₆]₂, [Cu₂(dppe)₃][NO₃]₂ [Cu(dcpb)Cl], [Cu(dcpb)Cl], [Cu(PPh₃)₂][NO₃] and [Cu(PCy₃)₂][NO₃], [CuCCPh] and [Cu₂C₂]*H₂O

If a preformed active catalyst is used, it can also be beneficial to add additional ligand of the formula I or V to the reaction mixture.

In the inventive process the amount of copper catalyst used based on the fromaldehyde used can be varied in a wide range. Usually the copper catalyst is used in a sub-stoichiometric amount relative to the formaldehyde. Typically, the amount of copper catalyst is not more than 50 mol%, frequently not more than 20 mol% and in particular not more than 10 mol% or not more than 5 mol%, based on the amount of formaldehyde. An amount of copper catalyst of from 0.001 to 50 mol%, frequently from 0.001 mol% to 20 mol% and in particular from 0.005 to 5 mol%, based on the amount of formaldehyde is preferably used in the process of the invention. Preference is given to using an amount of copper catalyst of from 0.01 to 5 mol%. All amounts of copper catalyst indicated are calculated as copper metal and based on the amount of formaldehyde.

The reaction of formaldehyde with acetylene can principally be performed according to all processes known to a person skilled in the art which are suitable for the reaction of formaldehyde with acetylene.

The acetylene used for the reduction reaction can be used in pure form or, if desired, also in the form of mixtures with other, preferably inert gases, such as nitrogen, methane, ethane, propane, butanes or argon. Preference is given to using acetylene in undiluted form.

The acetylene can be applied discontinuously or continuously, e.g. by bubbling acetylene gas through the reaction mixture.

The acetylene can also be applied to the reaction dissolved in an organic solvent. The solvent can be saturated with acetylene prior the reaction and then introduced to the alkynylation reaction dissolved in the organic solvent.

The process as described herein can also be used to produce substituted propargylic alcohols, e.g. phenylpropargylic alcohol or trimethylsilylpropargylic alcohol. For such a process accordingly substituted acetylene e.g. phenyl-acetylene or trimethylsilyl-acetylene is used instead of acetylene for the reaction with formaldehyde (see example 1-7 and example 8).

The reaction is typically carried at an acetylene pressure in the range from 0.1 to 20 bar, preferably in the range from 0.5 to 20 bar, more preferably in the range from 0.5 to 10 bar cold pressure.

In the method of the present invention, the reaction is carried out in the presence of an organic solvent. "Solvent" is a substance that dissolves a solute (a chemically different liquid, solid or gas), resulting in a solution. A solution is a homogenous mixture in which a gaseous, liquid or solid solute in dissolved in a solvent. A homogeneous mixture, in turn, is composed of two or more substances, where the particles of the solute cannot be seen by naked eye and which does not scatter light. In the present context, the solvent is liquid at 20°C.

The organic solvent is preferably selected from the group consisting of alkanes, cycloalkanes, aromatics, halogenated alkanes, halogenated aromatics.

Examples for suitable organic solvents are benzene, toluene, xylenes (mixtures of isomers and pure isomers), chloroform, (cyclo)-hexane, heptane or chlorobenzene

The formaldehyde is preferably present in an amount of from 0.1 to 25% by weight, more preferably from 0.5 to 20% by weight, in particular from 1 to 20% by weight, e.g. 1 to 15% by weight, relative to the overall weight of all solvents used in the alkynylation reaction.

Work-up of the reaction mixture of the inventive process and the isolation of the propargylic alcohol can of course also be effected in another customary manner, for example by filtration or aqueous extractive work-up, or distillation. The propargylic alcohol is generally obtained in sufficient purity by applying such measures or a combination thereof, obviating additional purification steps. Remaining copper catalyst e.g. in the sump of a distillation or remaining in the organic phase after aqueous extraction, can be recycled to the alkynylation reaction.

In one embodiment, the reaction is carried out in the presence of water, forming a second liquid phase in the reaction and also after the reaction. In this embodiment, one of the above-mentioned organic solvents is used, which are providing a mixing gap with water. The water can be added as pure water or via an aqueous formaldehyde solution, if aqueous formaldehyde is used as the formaldehyde source. In this embodiment, the copper-catalyst is preferably dissolved in the organic solvent and the propargylic alcohol is preferably dissolved in the aqueous phase after the reaction.

The ideal mixing ratio of organic solvent and water depends on the specific organic solvent, formaldehyde source used, catalysts, their respective concentrations and the phase separation method and can be determined by those skilled in the art.

Suitable amounts of organic solvent and water added in the alkynylation reaction are present in an overall weight ratio of preferably from 95:5 to 5:95, more preferably from 80:20 to 20:80.

The two liquid phases are generally separated by gravimetric phase separation. This may be carried out using, for example, standard apparatuses and standard methods which are described, for example, in E. Müller et al., "Liquid-Liquid Extraction" in Ullmann's Encyclopedia of Industrial Chemistry, 2005, Wiley-VCH Verlag GmbH&Co KGaA, DOI: 10.1002/14356007.b03_06, chapter 3 "Apparatus".

On a laboratory scale, phase separation can for example be carried out in a separatory funnel; on industrial scale, counter-current extraction apparatuses, such as mixer-settler apparatuses, extraction columns, agitated extraction columns, continuous packed bed liquid-liquid extraction apparatuses and the like are suitable.

The copper catalyst being enriched in the organic solvent phase means a partition coefficient of the copper catalyst P = [concentration of copper catalyst in the organic phase]/ [concentration of copper catalyst in the aqueous phase] of >1. The partition coefficient is preferably >2 and particularly preferably > 5.

The propargylic alcohol enriched in the aqueous phase means a partition coefficient of the propargylic alcohol
*P* = [concentration of propargylic alcohol in the aqueous phase]/ [concentration of propargylic alcohol in the organic phase]
   of >1. The partition coefficient is preferably >2 and particularly preferably > 5.

The propargylic alcohol can be isolated from the aqueous phase. For example, the product can be obtained by distillation or extraction.

The organic phase with the copper catalyst can be reused into the alkynylation reaction as catalyst phase with a satisfactory conversion rate after the first turnover. Even after several turnovers the catalytic activity is still satisfactory.

The reaction can principally be performed continuously, semi-continuously or discontinuously. Preference is given to a continuous process.

The reaction can principally be performed in all reactors known to a person skilled in the art for this type of reaction and who will therefore select the reactors accordingly. Suitable reactors are described and reviewed in the relevant prior art e.g. K. Henkel, "Reactor Types and Their Industrial Applications", Ullmann's Encyclopedia of Industrial Chemistry, 2005, Wiley-VCH Verlag GmbH & Co. KGaA, chapter 3.3: "Reactors for gas-liquid reactions".

The inventive process can be performed in a wide temperature range. Preferably the reaction is performed at a temperature in the range from 20 °C to 200 °C, more preferably in the range from 50°C to 180 °C, in particular in the range from 60°C to 120 °C.

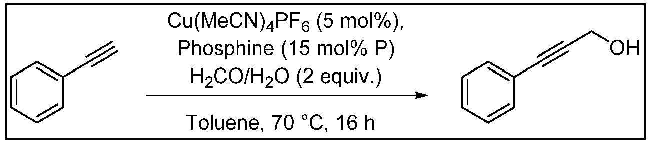

| **Example** | Phosphine | conv./**yield** (sel.) |
|---|---|---|
| **1** | - | 17%/**2%** (10%) |
| **2** | P(nBu)3 | 34%/**34%** (98%) |
| **3** | 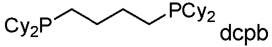 | 90%/**88%** (99%) |
| **4** | 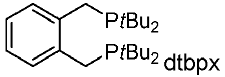 | 50%/**48%** (97%) |
| **5** | P(Cy)3 | 69%/**64%** (93%) |
| **6** | P(nOct)3 | 93%/**88%** (95%) |

### Experimental procedure for the ligand screening (examples 1-6):

Inside a argon filled Glove Box, a crimp vial (10 mL volume, single use glass vial with beaded rim, sealed with crimpable aluminum caps with Teflon/butyl rubber septum seal) was charged with tetrakis(acetonitrile)copper(I) hexafluorophosphate (18.6 mg, 50.0 µmol, 5.00 mol%), and ligand (monodentate: 150 µmol, 15.0 mol%; bidentate: 75.0 µmol, 7.50 mol%; for example: Tributylphosphine, 30.3 mg, 150 µmol, 15.0 mol%). The catalyst system was then dissolved in absolute toluene (5.00 mL, 47.2 mmol, 47.2 equiv.) and phenylacetylene (102 mg, 1.00 mmol, 1.00 equiv.) was added. A Teflon coated magnetic stirring bar was added, the vial was sealed and the solution was stirred for a few minutes at room temperature. The reaction mixture was then transferred outside of the glovebox and formaldehyde solution (ca. 30 *wt* in H₂O, trace amount of methanol, 0.200 mL, 2.00 mmol, 2.00 equiv.) was added *via* syringe through the septum of the reaction container. The reaction mixture was then stirred and heated by a metal heating block for 16 h at 70 °C. The vial was then cooled to room temperature, the reaction mixture was diluted with ethyl acetate (ca. 10 mL) and washed twice with ammonium hydroxide solution (ca. 10 mL) and once with brine (ca. 10 mL). The organic phase was then dried over MgSO₄, filtered and mesitylene (30.0 µL) was added as an internal GC standard. The reaction mixture was then analyzed by calibrated GC (t_{R}(phenylacetylene) = 5.70 min; t_{R}(phenylpropargylalcohol) = 10.83 min).

Analysis was done on an Agilent Technologies 6890N gas chromatograph with a split/splitless injector and an FID detector. The column used was an Agilent Technologies DB-1 capillary column (30 m*0.25 mm, 1 µm) with Helium as carrier gas.

GC method: Split: 50/1, 1.1 mL/min, const. pressure, 80 °C-1 min-15 °C/min-250 °C-5 min.

The comparative example 1 shows, that without a phosphine ligand, only traces of the product are formed. The examples 2 to 6 are showing, that by adding a phosphine ligand, the propargylic alcohol is formed as the main product.

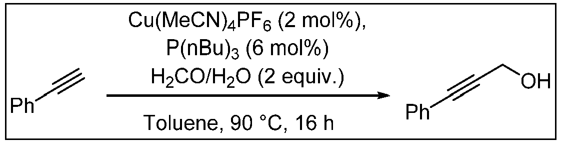

### Example 7: GC areas: starting material/product: 4/96

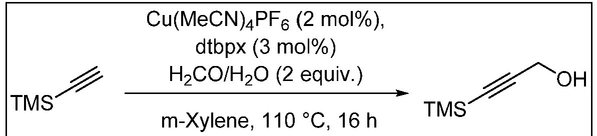

### Example 8: GC areas: starting material/product: 22/78

### Experimental procedure for the ethynylation of formaldehyde with acetylene derivatives (examples 7 and 8):

Inside a argon filled Glove Box, a crimp vial (10 mL volume, single use glass vial with beaded rim, sealed with crimpable aluminum caps with Teflon/butyl rubber septum seal) was charged with tetrakis(acetonitrile)copper(I) hexafluorophosphate (3.7 mg, 10.0 µmol, 2.00 mol%), and phosphine ligand (Tributylphosphine: 6.1 mg, 30.0 µmol, 6.00 mol% or 1,2-bis(di-*tert-*butylphosphino)xylene: 5.9 mg, 15.0 µmol, 3.00 mol%). The catalyst system was then dissolved in abs. toluene (5.00 mL, 47.2 mmol, 94.4 equiv.) or m-xylene (5.00 mL, 41.0 mmol, 82.0 equiv.) and phenylacetylene (51.1 mg, 500 µmol, 1.00 equiv.) or trimethylsilylacetylene (49.1 mg, 500 µmol, 1.00 equiv.) was added. A Teflon coated magnetic stirring bar was added, the vial was sealed and the solution was stirred and heated to 90 °C for a few minutes. The reaction mixture was then cooled to room temperature, transferred outside of the glovebox and formaldehyde solution (ca. 30 *wt* in H₂O, trace amount of methanol, 0.100 mL, 1.00 mmol, 2.00 equiv.) was added *via* syringe through the septum of the reaction container. The reaction mixture was then stirred and heated by a metal heating block for 16 h at 90 °C resp. 110 °C. The vial was then cooled to room temperature and the reaction mixture was analyzed by GC (t_{R}(phenylacetylene) = 3.52 min; t_{R}(phenylpropargylalcohol) = 9.50 min; t_{R}(TMS-acetylene) = 1.39 min; t_{R}(TMS-propargylalcohol) = 4.83 min).

Analysis was done on an Agilent Technologies 6890N gas chromatograph with a split/splitless injector and an FID detector. The column used was an Agilent Technologies DB-FFAP capillary column (30 m*0.32 mm, 0.25 µm) with Helium as carrier gas.

GC method: Split: 50/1, 2.3 mL/min, const. pressure, 80 °C-1 min-20 °C/min-250 °C-5 min.

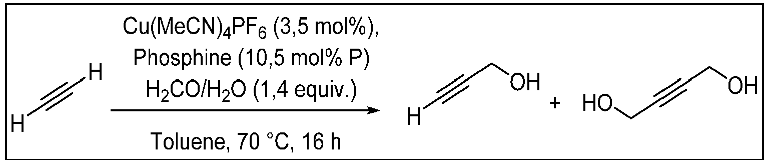

| **Example** | Phosphine | **GC yield OH** | GC yield 2OH |
|---|---|---|---|
| **9** | 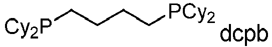 | **43%** | 10% |
| **10** | 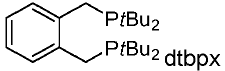 | **25%** | 12% |
| **11** | P(Cy)3 | **10%** | no |
| **12** | P(nOct)3 | **12%** | no |

### Experimental procedure for the ligand screening with acetylene (examples 9 -12):

Inside a Glove Box (Ar), a crimp vial (10 mL volume, single use glass vial with beaded rim, sealed with crimpable aluminum caps with Teflon/butyl rubber septum seal) was charged with tetrakis(acetonitrile)copper(I) hexafluorophosphate (6.5 mg, 17.5 µmol, 3.50 mol%), and phosphine ligand (monodentate: 52.5 µmol, 10.5 mol%; bidentate: 35.0 µmol, 7.00 mol%; for example: Tributylphosphine, 10.6 mg, 52.5 µmol, 10.5 mol%). The catalyst system was then dissolved in abs. solvent (5.00 mL) and a Teflon coated magnetic stirring bar was added. The vial was sealed and the solution was stirred for a few minutes at room temperature. The reaction mixture was then transferred outside of the glovebox and charged with acetylene by bubbling a stream of solvent-free and molecular sieve 3 Å dried acetylene *via* cannula for 2 minutes through the solution (acetylene concentration reaches saturation for atmospheric pressure, saturation concentration determined beforehand by calibrated GC, concentration of acetylene in toluene ca. 0.100 M, 0.500 mmol, 1.00 equiv.). The reaction mixture was then stirred for additional 10 minutes at room temperature and formaldehyde solution (ca. 30 *wt* in H₂O, trace amount of methanol, 70.1 µL, 0.700 mmol, 1.40 equiv.) was added *via* syringe through the septum of the reaction container. The reaction mixture was then stirred and heated by a metal heating block for 16 h at 70 °C. The vial was then cooled to room temperature, the reaction mixture was diluted with water (ca. 2 mL) and stirred for an additional 10 minutes. The water phase was then separated, filtered and DMSO (30.0 µL) was added as an internal GC standard. The water phase was then analyzed by calibrated GC (t_{R}(propargyl alcohol) = 2.59 min; t_{R}(butyne diol) = 6.75 min).

Analysis was done on an Agilent Technologies 6890N gas chromatograph with a split/splitless injector and an FID detector. The column used was an Agilent Technologies DB-1 capillary column (30 m*0.25 mm, 1 µm) with Helium as carrier gas.

GC method: Split: 50/1, 1.1 mL/min, const. pressure, 80 °C-1 min-15 °C/min-250 °C-5 min.

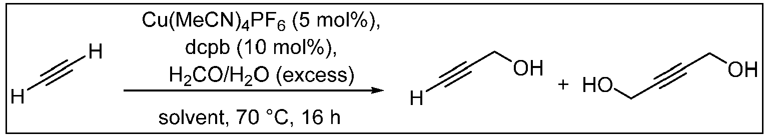

| **Example** | Solvent | c(Acetylene) | **GC yield OH** | GC yield 2OH |
|---|---|---|---|---|
| **13** | m-Xylene | 0,075 M (0,23 *wt*) | **52%** | 47% |
| **14** | Benzene | 0,1 M (0,30 *wt*) | **42%** | 45% |
| **15** | CHCl3 | 0,11 M (0,19 *wt*) | **37%** | 3% |
| **16** | Cyclohexane | 0,05 M (0,17 *wt*) | **23%** | 12% |
| **17** | Chlorbenzene | 0,09 M (0,21 *wt*) | **39%** | 14% |
| **18** | Toluene | 0,1 M (0,30 *wt*) | **43%** | 21% |

### Experimental procedure for the solvent screening (examples 13 to 18):

Inside a argon filled Glove Box, a crimp vial (10 mL volume, single use glass vial with beaded rim, sealed with crimpable aluminum caps with Teflon/butyl rubber septum seal) was charged with tetrakis(acetonitrile)copper(I) hexafluorophosphate (amount determined by the concentration of acetylene in solvent, example for toluene (0.100 M C₂H₂, 0.500 mmol C₂H₂, 1.00 equiv.): 9.3 mg, 25.0 µmol, 5.00 mol%), and 1,4-bis(dicyclohexylphosphino)butane (amount determined by the concentration of acetylene in solvent, example for toluene (0.100 M C₂H₂, 0.500 mmol C₂H₂, 1.00 equiv.): 22.5 mg, 50.0 µmol, 10.0 mol%). The catalyst system was then dissolved in abs. solvent (5.00 mL) and a Teflon coated magnetic stirring bar was added. The vial was sealed and the solution was stirred for a few minutes at room temperature. The reaction mixture was then transferred outside of the glovebox and charged with acetylene by bubbling a stream of solvent-free and molecular sieve 3 Å dried acetylene *via* cannula for 2 minutes through the solution (acetylene concentration reaches saturation for atmospheric pressure, saturation concentration determined beforehand by calibrated GC). The reaction mixture was then stirred for additional 10 minutes at room temperature and formaldehyde solution (ca. 30 *wt* in H₂O, trace amount of methanol, 0.100 mL, 1.00 mmol, 2.00 equiv.) was added *via* syringe through the septum of the reaction container. The reaction mixture was then stirred and heated by a metal heating block for 16 h at 70 °C. The vial was then cooled to room temperature, the reaction mixture was diluted with water (ca. 1 mL) and stirred for an additional 10 minutes. The water phase was then separated, filtered and DMSO (30.0 µL) was added as an internal GC standard. The water phase was then analyzed by calibrated GC (t_{R}(propargyl alcohol) = 2.59 min; t_{R}(butyne diol) = 6.75 min).

Analysis was done on an Agilent Technologies 6890N gas chromatograph with a split/splitless injector and an FID detector. The column used was an Agilent Technologies DB-1 capillary column (30 m*0.25 mm, 1 µm) with Helium as carrier gas.

GC method: Split: 50/1, 1.1 mL/min, const. pressure, 80 °C-1 min-15 °C/min-250 °C-5 min.

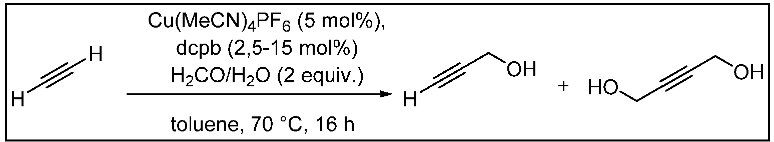

| **Example** | mol% ligand | x[Cu] | **GC yield OH** | GC yield 2OH |
|---|---|---|---|---|
| **19** | 7,5 | 1,5 | **38%** | 15% |
| **20** | 10 | 2 | **44%** | 29% |
| **21** | 15 | 3 | **44%** | 27% |

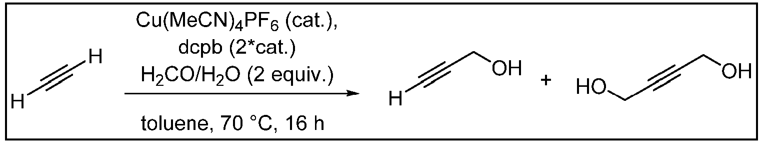

| **Example** | Catalyst loading [Cu]: | Catalyst loading [P]: | **GC yield OH**/yield 2OH |
|---|---|---|---|
| **22** | 5% | 10 mol% | **45%**/26% |
| **23** | 2% | 4 mol% | **46%**/18% |
| **24** | 1% | 2 mol% | **38%**/15% |

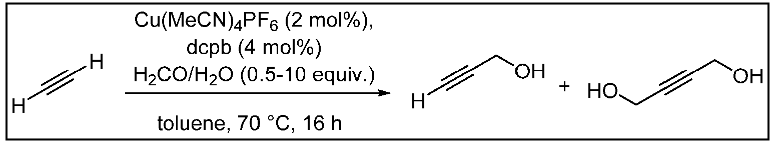

| **Example** | H₂CO equiv. | **GC yield OH**/yield 2OH |
|---|---|---|
| **25** | 1 | **52%**/9% |
| **26** | 2 | **57%**/40% |
| **27** | 5 | **57%**/29% |
| **28** | 10 | **40%**/5% |

| | | |
|---|---|---|
| (*calculated for H₂CO) | | |

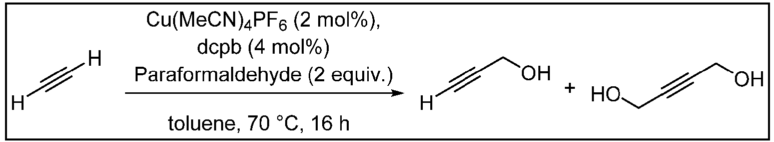

### Example 29: GC yield OH: 46%/2OH: 10%

### Experimental procedure for screening experiments with acetylene (examples 19-29):

Inside a argon filled Glove Box, a crimp vial (10 mL volume, single use glass vial with beaded rim, sealed with crimpable aluminum caps with Teflon/butyl rubber septum seal) was charged with tetrakis(acetonitrile)copper(I) hexafluorophosphate (0.2 mg - 9.3 mg, 0.500 - 25.0 µmol, 0.100 - 5.00 mol%), and 1,4-bis(dicyclohexylphosphino)butane (0.5 mg - 22.5 mg, 1.00 - 50.0 µmol, 0.200 - 10.0 mol%). The catalyst system was then dissolved in abs. toluene (5.00 mL, 47.2 mmol, 94.4 equiv.) and a Teflon coated magnetic stirring bar was added. The vial was sealed and the solution was stirred for a few minutes at room temperature. The reaction mixture was then filtered into a fresh crimp vial with a fresh Teflon coated magnetic stirring bar. If necessary, paraformaldehyde (30.0 mg, 1.00 mmol, 2.00 equiv.) was added. The vial was sealed and transferred outside of the glovebox. The reaction mixture was then charged with acetylene by bubbling a stream of solvent-free and molecular sieve 3 Å dried acetylene *via* cannula for 2 minutes through the solution (acetylene concentration reaches saturation for atmospheric pressure, saturation concentration determined beforehand by calibrated GC, concentration of acetylene in toluene ca. 0.100 M, 0.500 mmol, 1.00 equiv.). The reaction mixture was subsequently stirred for additional 10 minutes at room temperature and formaldehyde solution (ca. 30 *wt* in H₂O, trace amount of methanol, 25 - 500 µL, 0.250 - 5.00 mmol, 0.500 - 10.0 equiv.) was added, if necessary, *via* syringe through the septum of the reaction container. The reaction mixture was then stirred and heated by a metal heating block for 16 h at 70 °C. The vial was then cooled to room temperature, the reaction mixture was diluted with water (ca. 1 mL) and stirred for an additional 10 minutes. The water phase was then separated, filtered and DMSO (30.0 µL) was added as an internal GC standard. The water phase was then analyzed by calibrated GC (usually t_{R}(propargyl alcohol) = 3.90 min; t_{R}(butyne diol) = 8.60 min). Analysis was done on an Agilent Technologies 6890N gas chromatograph with a split/splitless injector and an FID detector. The column used was an Agilent Technologies DB-1 capillary column (30 m*0.25 mm, 1 µm) with Helium as carrier gas.

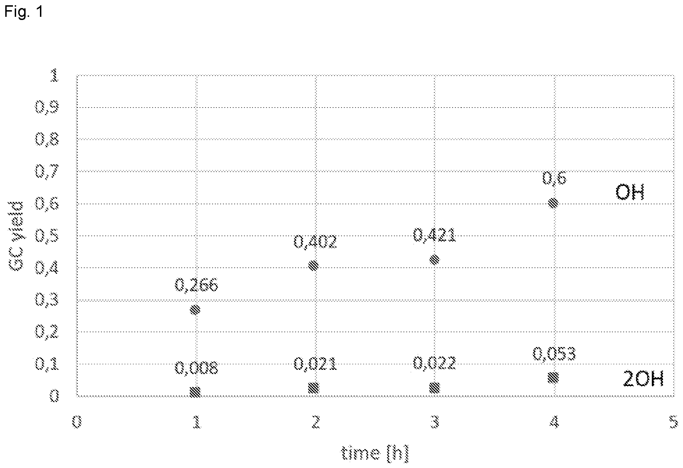

GC method: Split: 50/1, 1.1 mL/min, const. pressure, 80 °C-1 min-15 °C/min-250 °C-5 min. The results are shown in Fig. 1.

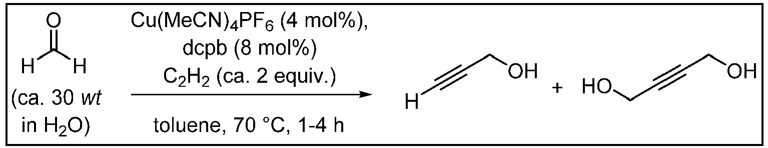

### Experimental procedure for the kinetic investigation (example 30):

Inside a Glove Box (Ar), a round-bottomed flask (50 mL volume) was charged with tetrakis(acetonitrile)copper(I) hexafluorophosphate (18.6 mg, 50.0 µmol, 4.00 mol%), and 1,4-bis(dicyclo-hexylphosphino)butane (45.1 mg, 100 µmol, 8.00 mol%). The catalyst system was then dissolved in abs. toluene (25.0 mL, 236 mmol, 189 equiv.) and a Teflon coated magnetic stirring bar was added. The solution was stirred for a few minutes at room temperature. The reaction mixture was then filtered into four crimp vials (10 mL volume, single use glass vial with beaded rim, sealed with crimpable aluminum caps with Teflon/butyl rubber septum seal) with Teflon coated magnetic stirring bars (5 mL each). The vials were sealed and transferred outside of the glovebox. The reaction mixtures were then charged with acetylene by bubbling a stream of solvent-free and molecular sieve 3 Å dried acetylene *via* cannula for 2 minutes through the solution (acetylene concentration reaches saturation for atmospheric pressure, saturation concentration determined beforehand by calibrated GC, concentration of acetylene in toluene ca. 0.100 M, 0.500 mmol, 2.00 equiv.). The reaction mixtures were subsequently stirred for additional 10 minutes at room temperature and formaldehyde solution (ca. 30 *wt* in H₂O, trace amount of methanol, 4x25.0 mg, 4x0.250 mmol, 4x1.00 equiv.) was added *via* syringe through the septum of each reaction container. The reaction mixtures were then stirred and heated by a metal heating block for 1 - 4 h at 70 °C. The vials were then cooled to room temperature, the reaction mixture was diluted with water (ca. 2 mL each) and then transferred into a separation funnel. The reaction vessel was washed with water (2x1 mL). The water phase was then separated and the organic phase was extracted two times with water (2 mL). The aqueous phases were collected and DMSO (30.0 µL) was added as an internal GC standard. The combined aqueous phase was then analyzed by calibrated GC (t_{R}(propargyl alcohol) = 3.40 min; t_{R}(butyne diol) = 9.15 min).

Analysis was done on an Agilent Technologies 6890N gas chromatograph with a split/splitless injector and an FID detector. The column used was an Agilent Technologies DB-FFAP capillary column (30 m*0.32 mm, 0.25 µm) with Helium as carrier gas.

GC method: Split: 50/1, 2.3 mL/min, const. pressure, 80 °C-1 min-20 °C/min-250 °C-5 min.

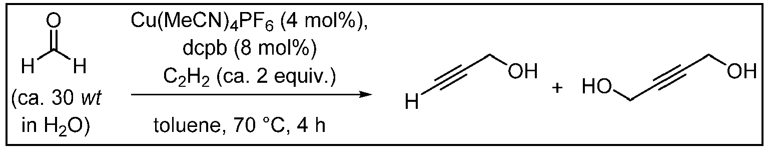

### Example 31: First run: GC yield OH/yield 2OH: 60%/5% second run: GC yield OH/yield 2OH: 59%/8%

### Experimental procedure for the catalyst recycling (example 31):

The organic phase obtained by the 4 h kinetic experiment *(vide supra)* was placed into a crimp vial (10 mL volume, single use glass vial with beaded rim, sealed with crimpable aluminum caps with Teflon/butyl rubber septum seal) with a Teflon coated magnetic stirring bar. The vial was sealed and the reaction mixture was then charged with acetylene by bubbling a stream of solvent-free and molecular sieve 3 Å dried acetylene *via* cannula for 2 minutes through the solution (acetylene concentration reaches saturation for atmospheric pressure, saturation concentration determined beforehand by calibrated GC, concentration of acetylene in toluene ca. 0.100 M, 0.500 mmol, 2.00 equiv.). Formaldehyde solution (ca. 30 *wt* in H₂O, trace amount of methanol, 25.0 mg, 0.250 mmol, 1.00 equiv.) was then added *via* syringe through the septum of the reaction container. The reaction mixture was then stirred and heated by a metal heating block for 4 h at 70 °C. The vial was then cooled to room temperature, the reaction mixture was diluted with water (ca. 2 mL) and then transferred into a separation funnel. The reaction vessel was washed with water (2x1 mL). The water phase was then separated and the organic phase was extracted two times with water (2 mL). The aqueous phases were collected and DMSO (30.0 µL) was added as an internal GC standard. The combined aqueous phase was then analyzed by calibrated GC (t_{R}(propargyl alcohol) = 3.40 min; t_{R}(butyne diol) = 9.15 min). Furthermore, both the aqueous phase and the organic phase were subjected to inductively coupled plasma mass spectrometry (ICP MS), performed in the certified central analytical department of BASF SE in Ludwigshafen, Germany.

Analysis was done on an Agilent Technologies 6890N gas chromatograph with a split/splitless injector and an FID detector. The column used was an Agilent Technologies DB-FFAP capillary column (30 m*0.32 mm, 0.25 µm) with Helium as carrier gas.

GC method: Split: 50/1, 2.3 mL/min, const. pressure, 80 °C-1 min-20 °C/min-250 °C-5 min. ICP-MS result: [Cu] in toluene: 15 mg/kg; [Cu] in H₂O: < 1 mg/kg.

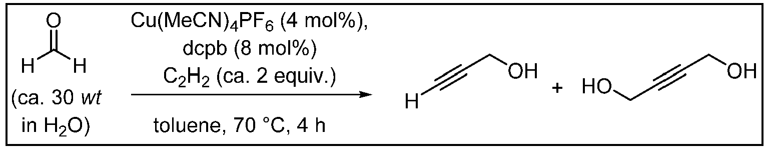

### Example 32: Experiment preparation under ambient conditions, GC yield OH/yield 2OH: 62%/2%

### Experimental procedure for the ethinylation of formaldehyde under ambient conditions (example 32):

A crimp vial (10 mL volume, single use glass vial with beaded rim, sealed with crimpable aluminum caps with Teflon/butyl rubber septum seal) was charged with tetrakis(acetonitrile)copper(I) hexafluorophosphate (3.7 mg, 10.0 µmol, 4.00 mol%), and 1,4-bis(dicyclohexylphosphino)butane (9.0 mg, 20.0 µmol, 8.00 mol%). The catalyst system was then dissolved in toluene (5.00 mL, 47.2 mmol, 189 equiv.) and a Teflon coated magnetic stirring bar was added. The solution was stirred for a few minutes at room temperature. The reaction mixture was then filtered into a fresh crimp vial with fresh Teflon coated magnetic stirring bar. The vial was then sealed and the reaction mixture was charged with acetylene by bubbling a stream of solvent-free and molecular sieve 3 Å dried acetylene *via* cannula for 2 minutes through the solution (acetylene concentration reaches saturation for atmospheric pressure, saturation concentration determined beforehand by calibrated GC, concentration of acetylene in toluene ca. 0.100 M, 0.500 mmol, 2.00 equiv.). Formaldehyde solution (ca. 30 *wt* in H₂O, trace amount of methanol, 25.0 mg, 0.250 mmol, 1.00 equiv.) was added *via* syringe through the septum of the reaction container. The reaction mixture was then stirred and heated by a metal heating block for 4 h at 70 °C. The vial was then cooled to room temperature, the reaction mixture was diluted with water (ca. 2 mL) and then transferred into a separation funnel. The reaction vessel was washed with water (2x1 mL). The water phase was then separated and the organic phase was extracted two times with water (2 mL). The aqueous phases were collected and DMSO (30.0 µL) was added as an internal GC standard. The combined aqueous phase was then analyzed by calibrated GC (t_{R}(propargyl alcohol) = 3.40 min; t_{R}(butyne diol) = 9.15 min).

Analysis was done on an Agilent Technologies 6890N gas chromatograph with a split/splitless injector and an FID detector. The column used was an Agilent Technologies DB-FFAP capillary column (30 m*0.32 mm, 0.25 µm) with Helium as carrier gas.

GC method: Split: 50/1, 2.3 mL/min, const. pressure, 80 °C-1 min-20 °C/min-250 °C-5 min.

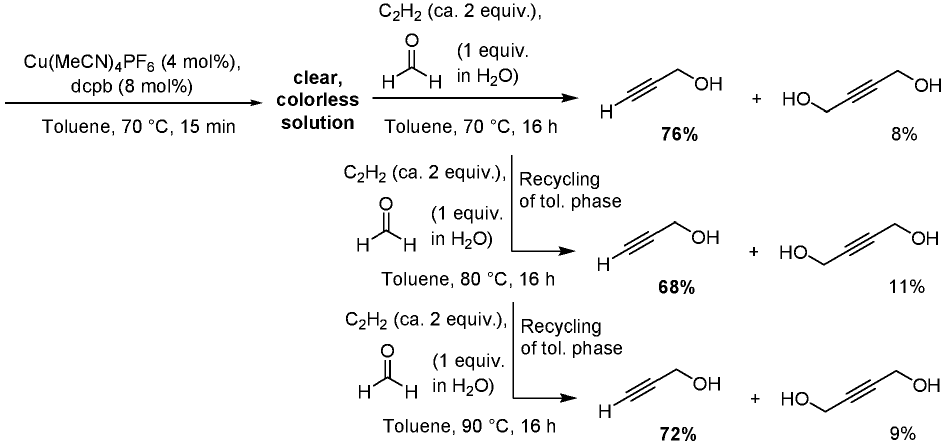

### (Yields determined by calibrated GC)

### Experimental procedure for the catalyst recycling (example 33):

Inside a Glove Box (Ar), a crimp vial (10 mL volume, single use glass vial with beaded rim, sealed with crimpable aluminum caps with Teflon/butyl rubber septum seal) was charged with tetrakis(acetonitrile)copper(I) hexafluorophosphate (3.7 mg, 10.0 µmol, 4.00 mol%), and 1,4-bis(dicyclohexylphosphino)butane (9.0 mg, 20.0 µmol, 8.00 mol%). The catalyst system was then dissolved in toluene (5.00 mL, 47.2 mmol, 189 equiv.) and a Teflon coated magnetic stirring bar was added. The solution was stirred for a few minutes at room temperature. The reaction mixture was then filtered into a fresh crimp vial with fresh Teflon coated magnetic stirring bar. The vial was then sealed and transferred outside the Glove Box. The reaction mixture was then heated to 70 °C for 15 min, cooled to room temperature and afterwards charged with acetylene by bubbling a stream of solvent-free and molecular sieve 3 Å dried acetylene *via* cannula for 2 minutes through the solution (acetylene concentration reaches saturation for atmospheric pressure, saturation concentration determined beforehand by calibrated GC, concentration of acetylene in toluene ca. 0.100 M, 0.500 mmol, 2.00 equiv.). Formaldehyde solution (ca. 30 *wt* in H₂O, trace amount of methanol, 25.0 mg, 0.250 mmol, 1.00 equiv.) was added *via* syringe through the septum of the reaction container. The reaction mixture was then stirred and heated by a metal heating block for 16 h at 70 °C. The vial was then cooled to room temperature, the reaction mixture was diluted with water (ca. 2 mL) and then transferred into a separation funnel. The reaction vessel was washed with water (2x1 mL). The water phase was then separated and the organic phase was extracted two times with water (2 mL). The aqueous phases were collected and DMSO (30.0 µL) was added as an internal GC standard. The combined aqueous phase was then analyzed by calibrated GC.

The organic phase was placed into a crimp vial (10 mL volume, single use glass vial with beaded rim, sealed with crimpable aluminum caps with Teflon/butyl rubber septum seal) with a Teflon coated magnetic stirring bar. The vial was sealed and the reaction mixture was then charged with acetylene by bubbling a stream of solvent-free and molecular sieve 3 Å dried acetylene *via* cannula for 2 minutes through the solution (acetylene concentration reaches saturation for atmospheric pressure, saturation concentration determined beforehand by calibrated GC, concentration of acetylene in toluene ca. 0.100 M, 0.500 mmol, 2.00 equiv.). Formaldehyde solution (ca. 30 *wt* in H₂O, trace amount of methanol, 25.0 mg, 0.250 mmol, 1.00 equiv.) was then added *via* syringe through the septum of the reaction container. The reaction mixture was then stirred and heated by a metal heating block for 16 h at 80 °C. The vial was then cooled to room temperature, the standard workup procedure was performed *(vide supra)* and the combined aqueous phases were, after addition of DMSO (30.0 µL) as internal GC standard, analyzed by calibrated GC.

The organic phase was once more placed into a crimp vial (10 mL volume, single use glass vial with beaded rim, sealed with crimpable aluminum caps with Teflon/butyl rubber septum seal) with a Teflon coated magnetic stirring bar. The vial was sealed and the reaction mixture was then charged with acetylene by bubbling a stream of solvent-free and molecular sieve 3 Å dried acetylene *via* cannula for 2 minutes through the solution (acetylene concentration reaches saturation for atmospheric pressure, saturation concentration determined beforehand by calibrated GC, concentration of acetylene in toluene ca. 0.100 M, 0.500 mmol, 2.00 equiv.). Formaldehyde solution (ca. 30 *wt* in H₂O, trace amount of methanol, 25.0 mg, 0.250 mmol, 1.00 equiv.) was then added *via* syringe through the septum of the reaction container. The reaction mixture was then stirred and heated by a metal heating block for 16 h at 90 °C. The vial was then cooled to room temperature, the standard workup procedure was performed *(vide supra)* and the combined aqueous phases were, after addition of DMSO (30.0 µL) as internal GC standard, analyzed by calibrated GC.

Analysis was done on an Agilent Technologies 6890N gas chromatograph with a split/splitless injector and an FID detector. The column used was an Agilent Technologies DB-FFAP capillary column (30 m*0.32 mm, 0.25 µm) with Helium as carrier gas.

GC method: Split: 50/1, 2.3 mL/min, const. pressure, 80 °C-1 min-20 °C/min-250 °C-5 min.

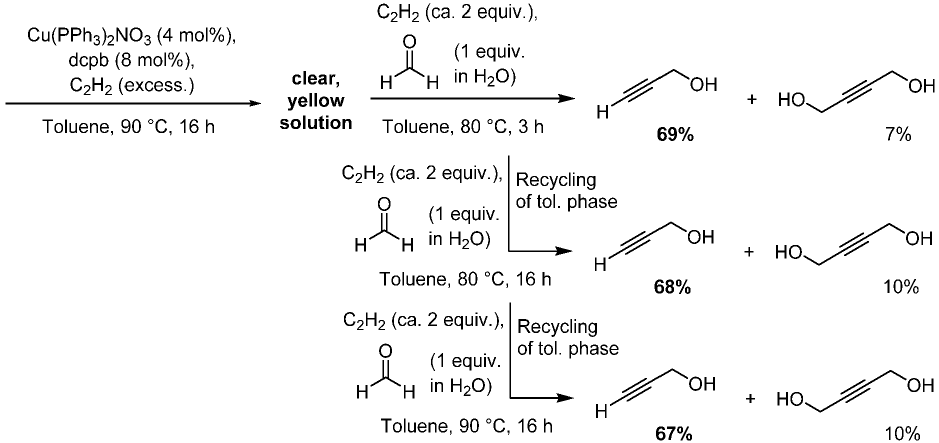

### (Yields determined by calibrated GC)

### Experimental procedure for the catalyst recycling with Bis(triphenylphosphine)copper(I) nitrate (example 34):

Inside a Glove Box (Ar), a crimp vial (10 mL volume, single use glass vial with beaded rim, sealed with crimpable aluminum caps with Teflon/butyl rubber septum seal) was charged with bis(triphenylphosphine)copper(I) nitrate (6.5 mg, 10.0 µmol, 4.00 mol%), and 1,4-bis(dicyclohexylphosphino)butane (9.0 mg, 20.0 µmol, 8.00 mol%). The catalyst system was then dissolved in toluene (5.00 mL, 47.2 mmol, 189 equiv.) and a Teflon coated magnetic stirring bar was added. The solution was stirred for a few minutes at room temperature. The vial was then sealed and transferred outside the Glove Box. The reaction mixture was then charged with acetylene by bubbling a stream of solvent-free and molecular sieve 3 Å dried acetylene *via* cannula for 2 minutes through the solution (acetylene concentration reaches saturation for atmospheric pressure, saturation concentration determined beforehand by calibrated GC, concentration of acetylene in toluene ca. 0.100 M, 0.500 mmol, 2.00 equiv.). The reaction mixture was then heated to 90 °C for 16 h. After cooling to room temperature, the reaction mixture was then charged once more with acetylene *(vide supra).* Formaldehyde solution (ca. 30 *wt* in H₂O, trace amount of methanol, 25.0 mg, 0.250 mmol, 1.00 equiv.) was then added *via* syringe through the septum of the reaction container. The reaction mixture was then stirred and heated by a metal heating block for 3 h at 80 °C. The vial was then cooled to room temperature, the reaction mixture was diluted with water (ca. 2 mL) and then transferred into a separation funnel. The reaction vessel was washed with water (2x1 mL). The water phase was then separated and the organic phase was extracted two times with water (2 mL). The aqueous phases were collected and DMSO (30.0 µL) was added as an internal GC standard. The combined aqueous phase was then analyzed by calibrated GC.

The organic phase was placed into a crimp vial (10 mL volume, single use glass vial with beaded rim, sealed with crimpable aluminum caps with Teflon/butyl rubber septum seal) with a Teflon coated magnetic stirring bar. The vial was sealed and the reaction mixture was then charged with acetylene by bubbling a stream of solvent-free and molecular sieve 3 Å dried acetylene *via* cannula for 2 minutes through the solution (acetylene concentration reaches saturation for atmospheric pressure, saturation concentration determined beforehand by calibrated GC, concentration of acetylene in toluene ca. 0.100 M, 0.500 mmol, 2.00 equiv.). Formaldehyde solution (ca. 30 *wt* in H₂O, trace amount of methanol, 25.0 mg, 0.250 mmol, 1.00 equiv.) was then added *via* syringe through the septum of the reaction container. The reaction mixture was then stirred and heated by a metal heating block for 16 h at 80 °C. The vial was then cooled to room temperature, the standard workup procedure was performed *(vide supra)* and the combined aqueous phases were, after addition of DMSO (30.0 µL) as internal GC standard, analyzed by calibrated GC.

The organic phase was once more placed into a crimp vial (10 mL volume, single use glass vial with beaded rim, sealed with crimpable aluminum caps with Teflon/butyl rubber septum seal) with a Teflon coated magnetic stirring bar. The vial was sealed and the reaction mixture was then charged with acetylene by bubbling a stream of solvent-free and molecular sieve 3 Å dried acetylene *via* cannula for 2 minutes through the solution (acetylene concentration reaches saturation for atmospheric pressure, saturation concentration determined beforehand by calibrated GC, concentration of acetylene in toluene ca. 0.100 M, 0.500 mmol, 2.00 equiv.). Formaldehyde solution (ca. 30 *wt* in H₂O, trace amount of methanol, 25.0 mg, 0.250 mmol, 1.00 equiv.) was then added *via* syringe through the septum of the reaction container. The reaction mixture was then stirred and heated by a metal heating block for 16 h at 90 °C. The vial was then cooled to room temperature, the standard workup procedure was performed *(vide supra)* and the combined aqueous phases were, after addition of DMSO (30.0 µL) as internal GC standard, analyzed by calibrated GC.

Analysis was done on an Agilent Technologies 6890N gas chromatograph with a split/splitless injector and an FID detector. The column used was an Agilent Technologies DB-FFAP capillary column (30 m*0.32 mm, 0.25 µm) with Helium as carrier gas.

GC method: Split: 50/1, 2.3 mL/min, const. pressure, 80 °C-1 min-20 °C/min-250 °C-5 min.

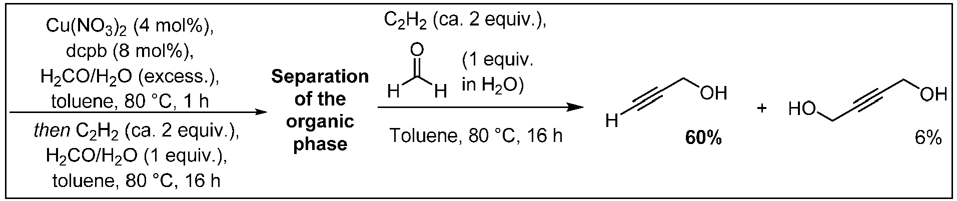

### (Yields determined by calibrated GC)

### Experimental procedure for the ethinylation of formaldehyde with copper(II) nitrate (example 35):

Inside a Glove Box (Ar), a crimp vial (10 mL volume, single use glass vial with beaded rim, sealed with crimpable aluminum caps with Teflon/butyl rubber septum seal) was charged with copper(II) nitrate trihydrate (2.4 mg, 10.0 µmol, 4.00 mol%), and 1,4-bis(dicyclohexylphosphino)butane (9.0 mg, 20.0 µmol, 8.00 mol%). The catalyst system was then dissolved in abs. toluene (5.00 mL, 47.2 mmol, 189 equiv.) and a Teflon coated magnetic stirring bar was added. The solution was stirred for a few minutes at room temperature. The vial was then sealed and transferred outside the Glove Box. Formaldehyde solution (ca. 30 *wt* in H₂O, trace amount of methanol, 25.0 mg, 0.250 mmol, 1.00 equiv.) was then added *via* syringe through the septum of the reaction container. The reaction mixture was then stirred and heated by a metal heating block for 1 h at 80 °C. After cooling to room temperature the reaction mixture was charged with acetylene by bubbling a stream of solvent-free and molecular sieve 3 Å dried acetylene *via* cannula for 2 minutes through the solution (acetylene concentration reaches saturation for atmospheric pressure, saturation concentration determined beforehand by calibrated GC, concentration of acetylene in toluene ca. 0.100 M, 0.500 mmol, 2.00 equiv.). Formaldehyde solution (ca. 30 *wt* in H₂O, trace amount of methanol, 25.0 mg, 0.250 mmol, 1.00 equiv.) was added *via* syringe through the septum of the reaction container and the reaction mixture was then heated to 80 °C for 16 h. After cooling to room temperature, the reaction mixture was diluted with water (ca. 2 mL) and then transferred into a separation funnel. The reaction vessel was washed with water (2x1 mL). The water phase was then separated and the organic phase was washed two times with water (2 mL). The organic phase was placed into a crimp vial (10 mL volume, single use glass vial with beaded rim, sealed with crimpable aluminum caps with Teflon/butyl rubber septum seal) with a Teflon coated magnetic stirring bar. The vial was sealed and the reaction mixture was then charged with acetylene *(vide supra).* Formaldehyde solution (ca. 30 *wt* in H₂O, trace amount of methanol, 25.0 mg, 0.250 mmol, 1.00 equiv.) was then added *via* syringe through the septum of the reaction container. The reaction mixture was then stirred and heated by a metal heating block for 16 h at 80 °C. The vial was then cooled to room temperature, the reaction mixture was diluted with water (ca. 2 mL) and then transferred into a separation funnel. The reaction vessel was washed with water (2x1 mL). The water phase was then separated and the organic phase was extracted two times with water (2 mL). and the combined aqueous phases were, after addition of DMSO (30.0 µL) as internal GC standard, analyzed by calibrated GC.

Analysis was done on an Agilent Technologies 6890N gas chromatograph with a split/splitless injector and an FID detector. The column used was an Agilent Technologies DB-FFAP capillary column (30 m*0.32 mm, 0.25 µm) with Helium as carrier gas.

GC method: Split: 50/1, 2.3 mL/min, const. pressure, 80 °C-1 min-20 °C/min-250 °C-5 min.

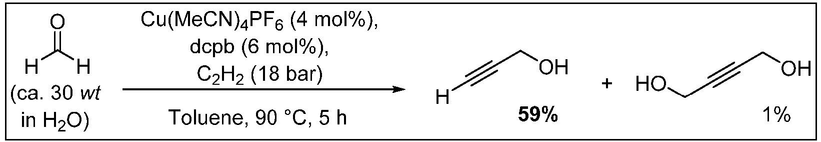

### (Yields determined by calibrated GC)

### Experimental procedure for the ethynylation with high acetylene pressure (example 36):

A round bottomed flask (500 mL volume) was charged with tetrakis(acetonitrile)copper(I) hexafluorophosphate (149 mg, 400 µmol, 4.00 mol%), and 1,4-bis(dicyclohexylphosphino)butane (270 mg, 600 µmol, 6.00 mol%). The catalyst system was then dissolved in toluene (200 mL, 1.89 mol, 189 equiv.) and a Teflon coated magnetic stirring bar was added. The solution was stirred for 5 minutes at room temperature and then decanted in a fresh round bottomed flask (500 mL volume) with Teflon coated stirring bar. The catalyst mixture was then heated to 80 °C by an oil bath and stirred for 30 minutes. The reaction mixture was then cooled to room temperature and transferred into a stainless steel autoclave (300 mL volume) equipped with a mechanical stirrer. Formaldehyde solution (ca. 30 *wt* in H₂O, trace amount of methanol, 1.00 mL, 10.0 mmol, 1.00 equiv.) was added and the autoclave was then sealed and pressurized at 90°C with acetylene (16 bar) and nitrogen (2 bar; 18 bar total pressure). The reaction mixture was stirred and heated for 5 h at 90 °C while preserving a constant acetylene pressure. The autoclave was then cooled to room temperature and depressurized. The reaction mixture was diluted with water (ca. 40 mL) and then transferred into a separation funnel. The reaction vessel was washed with water (2x40 mL). The water phase was then separated and the organic phase was extracted two times with water (40 mL each). The aqueous phases were collected and analyzed by calibrated GC.

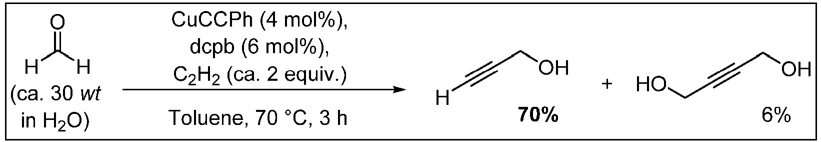

### (Yields determined by calibrated GC)

### Experimental procedure for the ethynylation of formaldehyde with copper(I) phenylacetylide (example 37):

Inside a Glove Box (Ar), a crimp vial (10 mL volume, single use glass vial with beaded rim, sealed with crimpable aluminum caps with Teflon/butyl rubber septum seal) was charged with copper phenylacetylide (1.6 mg, 10.0 µmol, 4.00 mol%), and 1,4-bis(dicyclohexylphosphino)butane (6.8 mg, 20.0 µmol, 6.00 mol%). The catalyst system was then dissolved in toluene (5.00 mL, 47.2 mmol, 189 equiv.) and a Teflon coated magnetic stirring bar was added. The vial was then sealed and transferred outside the Glove Box. The reaction mixture was then heated to 70 °C for 20 min until all solids were dissolved, cooled to room temperature and afterwards charged with acetylene by bubbling a stream of solvent-free and molecular sieve 3 Å dried acetylene *via* cannula for 2 minutes through the solution (acetylene concentration reaches saturation for atmospheric pressure, saturation concentration determined beforehand by calibrated GC, concentration of acetylene in toluene ca. 0.100 M, 0.500 mmol, 2.00 equiv.). Formaldehyde solution (ca. 30 *wt* in H₂O, trace amount of methanol, 25.0 mg, 0.250 mmol, 1.00 equiv.) was added *via* syringe through the septum of the reaction container. The reaction mixture was then stirred and heated by a metal heating block for 3 h at 70 °C. The vial was then cooled to room temperature, the reaction mixture was diluted with water (ca. 2 mL) and then transferred into a separation funnel. The reaction vessel was washed with water (2x1 mL). The water phase was then separated and the organic phase was extracted two times with water (2 mL). The aqueous phases were collected and DMSO (30.0 µL) was added as an internal GC standard. The combined aqueous phase was then analyzed by calibrated GC (t_{R}(propargyl alcohol) = 3.27 min; t_{R}(butyne diol) = 8.97 min).

Analysis was done on an Agilent Technologies 6890N gas chromatograph with a split/splitless injector and an FID detector. The column used was an Agilent Technologies DB-FFAP capillary column (30 m*0.32 mm, 0.25 µm) with Helium as carrier gas.

GC method: Split: 50/1, 2.3 mL/min, const. pressure, 80 °C-1 min-20 °C/min-250 °C-5 min.

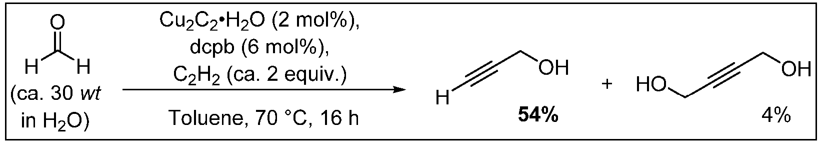

### (Yields determined by calibrated GC)

### Experimental procedure for the ethynylation of formaldehyde with copper(I) acetylide hydrate (example 38):

Inside a Glove Box (Ar), a crimp vial (10 mL volume, single use glass vial with beaded rim, sealed with crimpable aluminum caps with Teflon/butyl rubber septum seal) was charged with copper acetylide hydrate (0.8 mg, 5.00 µmol, 2.00 mol%), and 1,4-bis(dicyclohexylphosphino)butane (6.8 mg, 20.0 µmol, 6.00 mol%). The catalyst system was then dissolved in toluene (5.00 mL, 47.2 mmol, 189 equiv.) and a Teflon coated magnetic stirring bar was added. The vial was then sealed and transferred outside the Glove Box. The reaction mixture was then heated to 70 °C for 30 min, yielding a dark suspension. After cooling to room temperature, the vial was charged with acetylene by bubbling a stream of solvent-free and molecular sieve 3 Å dried acetylene *via* cannula for 2 minutes through the solution (acetylene concentration reaches saturation for atmospheric pressure, saturation concentration determined beforehand by calibrated GC, concentration of acetylene in toluene ca. 0.100 M, 0.500 mmol, 2.00 equiv.). Formaldehyde solution (ca. 30 *wt* in H₂O, trace amount of methanol, 25.0 mg, 0.250 mmol, 1.00 equiv.) was added *via* syringe through the septum of the reaction container. The reaction mixture was then stirred and heated by a metal heating block for 16 h at 70 °C. The vial was then cooled to room temperature, the reaction mixture was diluted with water (ca. 2 mL) and then transferred into a separation funnel. The reaction vessel was washed with water (2x1 mL). The water phase was then separated and the organic phase was extracted two times with water (2 mL). The aqueous phases were collected and DMSO (30.0 µL) was added as an internal GC standard. The combined aqueous phase was then analyzed by calibrated GC (t_{R}(propargyl alcohol) = 3.27 min; t_{R}(butyne diol) = 8.97 min).

Analysis was done on an Agilent Technologies 6890N gas chromatograph with a split/splitless injector and an FID detector. The column used was an Agilent Technologies DB-FFAP capillary column (30 m*0.32 mm, 0.25 µm) with Helium as carrier gas.

GC method: Split: 50/1, 2.3 mL/min, const. pressure, 80 °C-1 min-20 °C/min-250 °C-5 min.

## Claims

1. Process to produce propargylic alcohol, wherein acetylene is reacted with formaldehyde in the liquid phase in the presence of a copper catalyst and at least one phosphine.

2. Process according to claim 1, wherein the copper catalyst is a homogeneous catalyst comprising at least one phosphine as ligand.

3. Process according to claim 1 and 2 wherein the phosphine is a phosphine of formula I or II 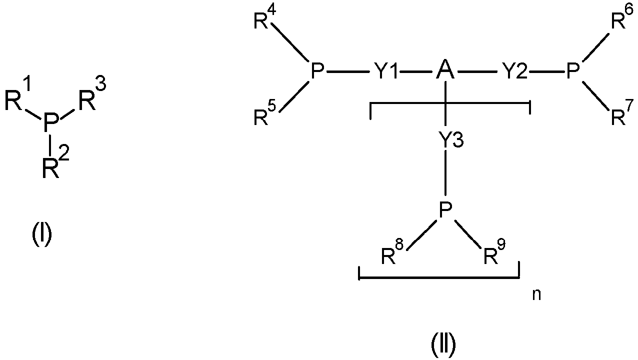 where
n is 0 or 1;
R¹ to R⁹ are, independently of one another, unsubstituted or at least monosubstituted C₁-C₁₀-alkyl, C₃-C₁₀-cycloalkyl, C₃-C₁₀-heterocyclyl comprising at least one heteroatom selected from N, O and S, C₅-C₁₄-aryl or C₅-C₁₀-heteroaryl comprising at least one heteroatom selected from N, O and S,
where the substituents are selected from the group consisting of: F, Cl, Br, OH, CN, NH₂ and C₁-C₁₀-alkyl;
A is
i) a bridging group selected from the group unsubstituted or at least monosubstituted N, O, P, C₁-C₆-alkane, C₃-C₁₀-cycloalkane, C₃-C₁₀-heterocycloalkane comprising at least one heteroatom selected from N, O and S, C₅-C₁₄-aromatic and C₅-C₆-heteroaromatic comprising at least one heteroatom selected from N, O and S, where the substituents are selected from the group consisting of:
C₁-C₄-alkyl, phenyl, F, Cl, Br, OH, OR¹⁵, NH₂, NHR¹⁵ or N(R¹⁵)₂,
where R¹⁵ is selected from C₁-C₁₀-alkyl and C₅-C₁₀-aryl;
or
ii) a bridging group of the formula (III) or (IV): 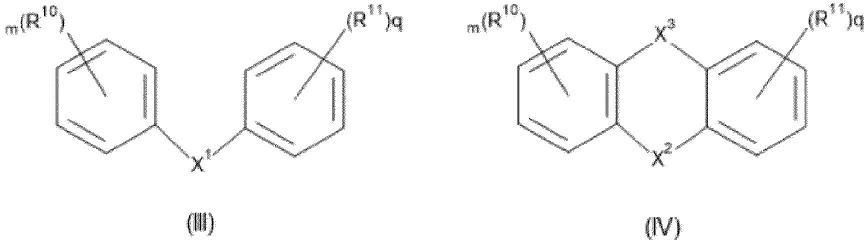
m, q are, independently of one another, 0, 1, 2, 3 or 4;
R¹⁰, R¹¹ are, independently of one another, selected from the group C₁-C₁₀-alkyl, F, Cl, Br, OH, OR¹⁸, NH₂, NHR¹⁸ and N(R¹⁸)₂,
where R¹⁸ is selected from C₁-C₁₀-alkyl and C₅-C₁₀-aryl;
X¹, X² are, independently of one another, NH, O or S;
X³ is a bond, NH, NR¹⁷, O, S or CR¹⁸R¹⁹;
R¹⁷ is unsubstituted or at least monosubstituted C₁-C₁₀-alkyl, C₃-C₁₀-cycloalkyl, C₃-C₁₀-heterocyclyl comprising at least one heteroatom selected from N, O and S, C₅-C₁₄-aryl or C₅-C₁₀-heteroaryl comprising at least one heteroatom selected from N, O and S,
where the substituents are selected from the group consisting of: F, Cl, Br, OH, CN, NH₂ and C₁-C₁₀-alkyl;
R¹⁸, R¹⁹ are, independently of one another, unsubstituted or at least monosubstituted C₁-C₁₀-alkyl, C₁-C₁₀-alkoxy, C₃-C₁₀-cycloalkyl, C₃-C₁₀-cycloalkoxy, C₃-C₁₀-heterocyclyl comprising at least one heteroatom selected from N, O and S, C₅-C₁₄-aryl, C₅-C₁₄-aryloxy or C₅-C₁₀-heteroaryl comprising at least one heteroatom selected from N, O and S,
where the substituents are selected from the group consisting of: F, Cl, Br, OH, CN, NH₂ and C₁-C₁₀-alkyl;
Y¹, Y², Y³ are, independently of one another, a bond, unsubstituted or at least monosubstituted methylene, ethylene, trimethylene, tetramethylene, pentamethylene or hexamethylene,
where the substituents are selected from the group consisting of: F, Cl, Br, OH, OR¹⁵, CN, NH₂, NHR¹⁶, N(R¹⁶)₂ and C₁-C₁₀-alkyl, where R¹⁶ is selected from C₁-C₁₀-alkyl and C₅-C₁₀-aryl.

4. Process according to claims 1 to 3, wherein the phosphine is a bidentate phosphine according formula V 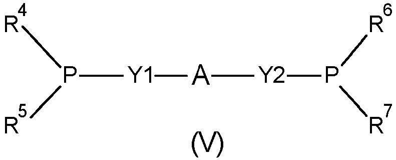 where
R⁴ to R⁷ are, independently of one another, unsubstituted or at least monosubstituted C₁-C₁₀-alkyl, C₃-C₁₀-cycloalkyl, C₃-C₁₀-heterocyclyl comprising at least one heteroatom selected from N, O and S, C₅-C₁₄-aryl or C₅-C₁₀-heteroaryl comprising at least one heteroatom selected from N, O and S,
where the substituents are selected from the group consisting of: F, Cl, Br, OH, CN, NH₂ and C₁-C₁₀-alkyl;
A is
i) a bridging group selected from the group unsubstituted or at least monosubstituted N, O, P, C₁-C₆-alkane, C₃-C₁₀-cycloalkane, C₃-C₁₀-heterocycloalkane comprising at least one heteroatom selected from N, O and S, C₅-C₁₄-aromatic and C₅-C₆-heteroaromatic comprising at least one heteroatom selected from N, O and S,
where the substituents are selected from the group consisting of:
C₁-C₄-alkyl, phenyl, F, Cl, Br, OH, OR¹⁶, NH₂, NHR¹⁶ or N(R¹⁶)₂,
where R¹⁶ is selected from C₁-C₁₀-alkyl and C₅-C₁₀-aryl;

5. Process according to any of claims 1 to 4, wherein the copper complex is prepared in situ during the reaction of the formaldehyde with acetylene.

6. Process according to claim 5, wherein 0.1 to 10 mol of phosphine per mol of copper are used in the preparation of the copper complex

7. Process according to any of claims 1 to 6, wherein the copper complex is used in an amount of 0.01 to 5 mol% based on the amount of formaldehyde.

8. Process according to any of claims 1 to 7, wherein the reaction mixture comprises an organic solvent.

9. Process according to any of claims 1 to 8, acetylene is fed to the reaction with a pressure of 1 to 20 bar.

10. Process according to any of claims 1 to 9, wherein the reaction is performed at a temperature of 50 to 200°C

11. Process according to any of claims 1 to 10, wherein the reaction is carried out in a system containing two liquid phases, wherein one phase is a water rich phase and the other enriched with an organic solvent.

12. Process according to claim 10, wherein the copper catalyst is enriched after the reaction in the organic phase and the propargylic alcohol is enriched in the aqueous phase.

13. Process according to claim 10 and 11, wherein the copper catalyst is enriched after the reaction in the organic phase is reused as catalyst in the alkynylation reaction.

## Patentansprüche

1. Verfahren zur Herstellung von Propargylalkohol, bei dem Acetylen in der Flüssigphase in Gegenwart eines Kupferkatalysators und mindestens eines Phosphins mit Formaldehyd umgesetzt wird.

2. Verfahren nach Anspruch 1, wobei es sich bei dem Kupferkatalysator um einen homogenen Katalysator handelt, der mindestens ein Phosphin als Liganden umfasst.

3. Verfahren nach Anspruch 1 und 2, wobei es sich bei dem Phosphin um ein Phosphin der Formel I oder II handelt: 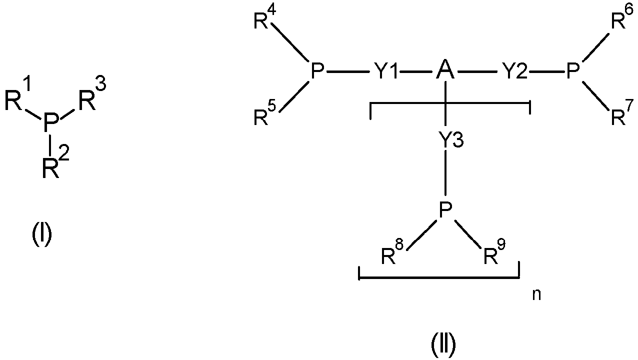 wobei
n für 0 oder 1 steht;
R¹ bis R⁹ unabhängig voneinander für unsubstituiertes oder zumindest monosubstituiertes C₁-C₁₀-Alkyl, C₃-C₁₀-Cycloalkyl, C₃-C₁₀-Heterocyclyl mit mindestens einem Heteroatom, das aus N, O und S ausgewählt ist, C₅-C₁₄-Aryl oder C₅-C₁₀-Heteroaryl mit mindestens einem Heteroatom, das aus N, O und S ausgewählt ist, stehen,
wobei die Substituenten ausgewählt sind aus der Gruppe bestehend aus: F, Cl, Br, OH, CN, NH₂ und C₁-C₁₀-Alkyl;
A für Folgendes steht:
i) eine Brückengruppe, ausgewählt aus der Gruppe unsubstituiertes oder zumindest monosubstituiertes N, O, P, C₁-C₆-Alkan, C₃-C₁₀-Cycloalkan, C₃-C₁₀-Heterocycloalkan mit mindestens einem Heteroatom, das aus N, O und S ausgewählt ist, C₅-C₁₄-Aromat und C₅-C₆-Heteroaromat mit mindestens einem Heteroatom, das aus N, O und S ausgewählt ist,
wobei die Substituenten ausgewählt sind aus der Gruppe bestehend aus:
C₁-C₄-Alkyl, Phenyl, F, Cl, Br, OH, OR¹⁵, NH₂, NHR¹⁵ oder N(R¹⁵)₂,
wobei R¹⁵ ausgewählt ist aus C₁-C₁₀-Alkyl und C₅-C₁₀-Aryl; oder
ii) eine Brückengruppe der Formel (III) oder (IV): 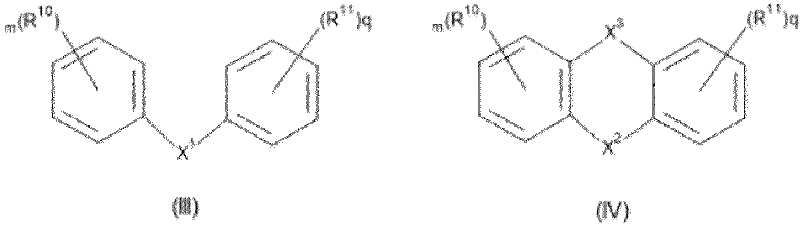 m, q unabhängig voneinander für 0, 1, 2, 3 oder 4 stehen; R¹⁰, R¹¹ unabhängig voneinander ausgewählt sind aus der Gruppe C₁-C₁₀-Alkyl, F, Cl, Br, OH, OR¹⁸, NH₂, NHR¹⁸ und N(R¹⁸)₂,
wobei R¹⁸ ausgewählt ist aus C₁-C₁₀-Alkyl und C₅-C₁₀-Aryl;
X¹, X² unabhängig voneinander für NH, O oder S stehen;
X³ für eine Bindung, NH, NR¹⁷, O, S oder CR¹⁸R¹⁹ steht;
R¹⁷ für unsubstituiertes oder zumindest monosubstituiertes C₁-C₁₀-Alkyl, C₃-C₁₀-Cycloalkyl, C₃-C₁₀-Heterocyclyl mit mindestens einem Heteroatom, das aus N, O und S ausgewählt ist, C₅-C₁₄-Aryl oder C₅-C₁₀-Heteroaryl mit mindestens einem Heteroatom, das aus N, O und S ausgewählt ist, steht,
wobei die Substituenten ausgewählt sind aus der Gruppe bestehend aus: F, Cl, Br, OH, CN, NH₂ und C₁-C₁₀-Alkyl;
R¹⁸, R¹⁹ unabhängig voneinander für unsubstituiertes oder zumindest monosubstituiertes C₁-C₁₀-Alkyl, C₁-C₁₀-Alkoxy, C₃-C₁₀-Cycloalkyl, C₃-C₁₀-Cycloalkoxy, C₃-C₁₀-Heterocyclyl mit mindestens einem Heteroatom, das aus N, O und S ausgewählt ist, C₅-C₁₄-Aryl, C₅-C₁₄-Aryloxy oder C₅-C₁₀-Heteroaryl mit mindestens einem Heteroatom, das aus N, O und S ausgewählt ist, stehen,
wobei die Substituenten ausgewählt sind aus der Gruppe bestehend aus: F, Cl, Br, OH, CN, NH₂ und C₁-C₁₀-Alkyl;
Y¹, Y², Y³ unabhängig voneinander für eine Bindung, unsubstituiertes oder zumindest monosubstituiertes Methylen, Ethylen, Trimethylen, Tetramethylen, Pentamethylen oder Hexamethylen stehen,
wobei die Substituenten ausgewählt sind aus der Gruppe bestehend aus: F, Cl, Br, OH, OR¹⁵, CN, NH₂, NHR¹⁶, N(R¹⁶)₂ und C₁-C₁₀-Alkyl,
wobei R¹⁶ ausgewählt ist aus C₁-C₁₀-Alkyl und C₅-C₁₀-Aryl.

4. Verfahren nach den Ansprüchen 1 bis 3, wobei es sich bei dem Phosphin um ein zweizähniges Phosphin gemäß Formel V handelt: 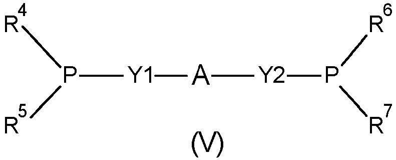 wobei
R⁴ bis R⁷ unabhängig voneinander für unsubstituiertes oder zumindest monosubstituiertes C₁-C₁₀-Alkyl, C₃-C₁₀-Cycloalkyl, C₃-C₁₀-Heterocyclyl mit mindestens einem Heteroatom, das aus N, O und S ausgewählt ist, C₅-C₁₄-Aryl oder C₅-C₁₀-Heteroaryl mit mindestens einem Heteroatom, das aus N, O und S ausgewählt ist, stehen,
wobei die Substituenten ausgewählt sind aus der Gruppe bestehend aus: F, Cl, Br, OH, CN, NH₂ und C₁-C₁₀-Alkyl;
A für Folgendes steht:
i) eine Brückengruppe, ausgewählt aus der Gruppe unsubstituiertes oder zumindest monosubstituiertes N, O, P, C₁-C₆-Alkan, C₃-C₁₀-Cycloalkan, C₃-C₁₀-Heterocycloalkan mit mindestens einem Heteroatom, das aus N, O und S ausgewählt ist, C₅-C₁₄-Aromat und C₅-C₆-Heteroaromat mit mindestens einem Heteroatom, das aus N, O und S ausgewählt ist,
wobei die Substituenten ausgewählt sind aus der Gruppe bestehend aus:
C₁-C₄-Alkyl, Phenyl, F, Cl, Br, OH, OR¹⁶, NH₂, NHR¹⁶ oder N(R¹⁶)₂,
wobei R¹⁶ ausgewählt ist aus C₁-C₁₀-Alkyl und C₅-C₁₀-Aryl;

5. Verfahren nach einem der Ansprüche 1 bis 4, wobei der Kupferkomplex während der Umsetzung des Formaldehyds mit Acetylen in situ hergestellt wird.

6. Verfahren nach Anspruch 5, wobei bei der Herstellung des Kupferkomplexes 0,1 bis 10 Mol Phosphin pro Mol Kupfer verwendet werden.

7. Verfahren nach einem der Ansprüche 1 bis 6, wobei der Kupferkomplex in einer Menge von 0,01 bis 5 Mol-%, bezogen auf die Menge an Formaldehyd, verwendet wird.

8. Verfahren nach einem der Ansprüche 1 bis 7, wobei die Reaktionsmischung ein organisches Lösungsmittel umfasst.

9. Verfahren nach einem der Ansprüche 1 bis 8, bei dem Acetylen der Umsetzung mit einem Druck von 1 bis 20 bar zugeführt wird.

10. Verfahren nach einem der Ansprüche 1 bis 9, wobei Umsetzung bei einer Temperatur von 50 °C bis 200 °C durchgeführt wird.

11. Verfahren nach einem der Ansprüche 1 bis 10, wobei die Umsetzung in einem System durchgeführt wird, das zwei Flüssigphasen enthält, wobei die eine Phase eine wasserreiche Phase ist und die andere mit einem organischen Lösungsmittel angereichert ist.

12. Verfahren nach Anspruch 10, wobei der Kupferkatalysator nach der Umsetzung in der organischen Phase angereichert ist und der Propargylalkohol in der wässrigen Phase angereichert ist.

13. Verfahren nach Anspruch 10 und 11, wobei der Kupferkatalysator nach der Umsetzung in der organischen Phase angereichert ist und als Katalysator bei der Alkinylierungsreaktion wiederverwendet wird.

## Revendications

1. Procédé de production d'alcool propargylique, dans lequel on fait réagir de l'acétylène avec du formaldéhyde dans la phase liquide en présence d'un catalyseur au cuivre et d'au moins une phosphine.

2. Procédé selon la revendication 1, dans lequel le catalyseur au cuivre est un catalyseur homogène comprenant au moins une phosphine comme ligand.

3. Procédé selon les revendications 1 et 2, dans lequel la phosphine est une phosphine de formule I ou II 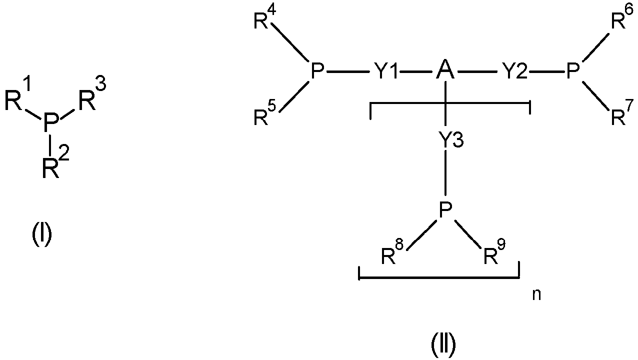 où
n est 0 ou 1 ;
R¹ à R⁹ sont, indépendamment l'un de l'autre, C₁-C₁₀-alkyle, C₃-C₁₀-cycloalkyle, C₃-C₁₀-hétérocyclyle non substitué ou au moins monosubstitué comprenant au moins un hétéroatome choisi parmi N, O et S, C₅-C₁₄-aryle ou C₅-C₁₀-hétéroaryle comprenant au moins un hétéroatome choisi parmi N, O et S,
où les substituants sont choisis dans le groupe constitué par : F, Cl, Br, OH, CN, NH₂ et C₁-C₁₀-alkyle ;
A est
i) un groupe pontant choisi dans le groupe N, O, P, C₁-C₆-alcane, C₃-C₁₀-cycloalcane, C₃-C₁₀-hétérocycloalcane non substitué ou au moins monosubstitué comprenant au moins un hétéroatome choisi parmi N, O et S, C₅-C₁₄-aromatique et C₅-C₆-hétéroaromatique comprenant au moins un hétéroatome choisi parmi N, O et S,
où les substituants sont choisis dans le groupe constitué par :
C₁-C₄-alkyle, phényle, F, Cl, Br, OH, OR¹⁵, NH₂, NHR¹⁵ ou N(R¹⁵)₂,
où R¹⁵ est choisi parmi C₁-C₁₀-alkyle et C₅-C₁₀-aryle ; ou
ii) un groupe pontant de la formule (III) ou (IV) : 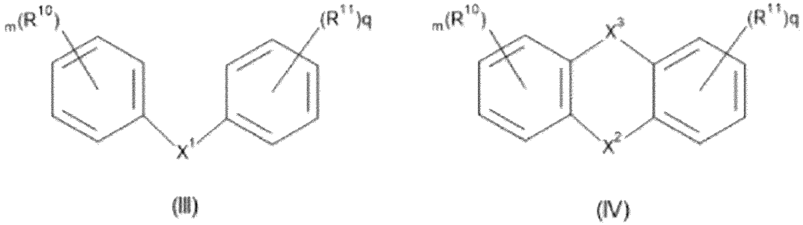 m, q sont, indépendamment l'un de l'autre, 0, 1, 2, 3 ou 4 ;
R¹⁰, R¹¹ sont, indépendamment l'un de l'autre, choisis dans le groupe C₁-C₁₀-alkyle, F, Cl, Br, OH, OR¹⁸, NH₂, NHR¹⁸ et N(R¹⁸)₂,
où R¹⁸ est choisi parmi C₁-C₁₀-alkyle et C₅-C₁₀-aryle ;
X¹, X² sont, indépendamment l'un de l'autre, NH, O ou S ; X³ est une liaison, NH, NR¹⁷, O, S ou CR¹⁸R¹⁹ ;
R¹⁷ est C₁-C₁₀-alkyle, C₃-C₁₀-cycloalkyle, C₃-C₁₀-hétérocyclyle non substitué ou au moins monosubstitué comprenant au moins un hétéroatome choisi parmi N, O et S, C₅-C₁₄-aryle ou C₅-C₁₀-hétéroaryle comprenant au moins un hétéroatome choisi parmi N, O et S,
où les substituants sont choisis dans le groupe constitué par : F, Cl, Br, OH, CN, NH₂ et C₁-C₁₀-alkyle ;
R¹⁸, R¹⁹ sont, indépendamment l'un de l'autre, C₁-C₁₀-alkyle, C₁-C₁₀-alcoxy, C₃-C₁₀-cycloalkyle, C₃-C₁₀-cycloalcoxy, C₃-C₁₀-hétérocyclyle non substitué ou au moins monosubstitué comprenant au moins un hétéroatome choisi parmi N, O et S, C₅-C₁₄-aryle, C₅-C₁₄-aryloxy ou C₅-C₁₀-hétéroaryle comprenant au moins un hétéroatome choisi parmi N, O et S,
où les substituants sont choisis dans le groupe constitué par : F, Cl, Br, OH, CN, NH₂ et C₁-C₁₀-alkyle ;
Y¹, Y², Y³ sont, indépendamment l'un de l'autre, une liaison, méthylène, éthylène, triméthylène, tétraméthylène, pentaméthylène ou hexaméthylène non substitué ou au moins monosubstitué,
où les substituants sont choisis dans le groupe constitué par : F, Cl, Br, OH, OR¹⁵, CN, NH₂, NHR¹⁶, N(R¹⁶)₂ et C₁-C₁₀-alkyle,
où R¹⁶ est choisi parmi C₁-C₁₀-alkyle et C₅-C₁₀-aryle.

4. Procédé selon les revendications 1 à 3, dans lequel la phosphine est une phosphine bidentate selon la formule V 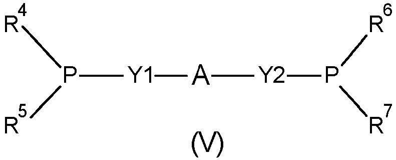 où
R⁴ à R⁷ sont, indépendamment l'un de l'autre, C₁-C₁₀-alkyle, C₃-C₁₀-cycloalkyle, C₃-C₁₀-hétérocyclyle non substitué ou au moins monosubstitué comprenant au moins un hétéroatome choisi parmi N, O et S, C₅-C₁₄-aryle ou C₅-C₁₀-hétéroaryle comprenant au moins un hétéroatome choisi parmi N, O et S,
où les substituants sont choisis dans le groupe constitué par : F, Cl, Br, OH, CN, NH₂ et C₁-C₁₀-alkyle ;
A est
i) un groupe pontant choisi dans le groupe N, O, P, C₁-C₆-alcane, C₃-C₁₀-cycloalcane, C₃-C₁₀-hétérocycloalcane non substitué ou au moins monosubstitué comprenant au moins un hétéroatome choisi parmi N, O et S, C₅-C₁₄-aromatique et C₅-C₆-hétéroaromatique comprenant au moins un hétéroatome choisi parmi N, O et S,
où les substituants sont choisis dans le groupe constitué par :
C₁-C₄-alkyle, phényle, F, Cl, Br, OH, OR¹⁶, NH₂, NHR¹⁶ ou N(R¹⁶)₂,
où R¹⁶ est choisi parmi C₁-C₁₀-alkyle et C₅-C₁₀-aryle .

5. Procédé selon l'une quelconque des revendications 1 à 4, dans lequel le complexe de cuivre est préparé *in situ* pendant la réaction du formaldéhyde avec l'acétylène.

6. Procédé selon la revendication 5, dans lequel on utilise 0,1 à 10 moles de phosphine par mole de cuivre pour la préparation du complexe de cuivre.

7. Procédé selon l'une quelconque des revendications 1 à 6, dans lequel le complexe de cuivre est utilisé en une quantité de 0,01 à 5 % en moles sur la base de la quantité de formaldéhyde.

8. Procédé selon l'une quelconque des revendications 1 à 7, dans lequel le mélange réactionnel comprend un solvant organique.

9. Procédé selon l'une quelconque des revendications 1 à 8, dans lequel de l'acétylène est alimenté dans la réaction avec une pression de 1 à 20 bars.

10. Procédé selon l'une quelconque des revendications 1 à 9, dans lequel la réaction est réalisée à une température de 50 à 200 °C.

11. Procédé selon l'une quelconque des revendications 1 à 10, dans lequel la réaction est effectuée dans un système contenant deux phases liquides, dans lequel une phase est une phase riche en eau et l'autre est enrichie avec un solvant organique.

12. Procédé selon la revendication 10, dans lequel le catalyseur au cuivre est enrichi après la réaction dans la phase organique et l'alcool propargylique est enrichi dans la phase aqueuse.

13. Procédé selon les revendications 10 et 11, dans lequel le catalyseur au cuivre est enrichi après la réaction dans la phase organique et réutilisé comme catalyseur dans la réaction d'alcynylation.
